(19)

**Europäisches Patentamt**
**European Patent Office**
**Office européen des brevets**

(11) **EP 4 279 093 A1**

(12) **EUROPEAN PATENT APPLICATION**

(43) Date of publication:
**22.11.2023 Bulletin 2023/47**

(21) Application number: **23382452.3**

(22) Date of filing: **12.05.2023**

(51) International Patent Classification (IPC):
**A61L 27/26** (2006.01)   **A61L 27/38** (2006.01)
**A61L 27/52** (2006.01)   **A61L 27/58** (2006.01)
**A61L 27/60** (2006.01)   **B33Y 80/00** (2015.01)

(52) Cooperative Patent Classification (CPC):
(C-Sets available)
**A61L 27/58; A61L 27/26; A61L 27/3804;**
**A61L 27/3813; A61L 27/3834; A61L 27/3843;**
**A61L 27/3886; A61L 27/52; A61L 27/60;**
**B33Y 80/00;** A61L 2430/34         (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **20.05.2022 ES 202230432**

(71) Applicants:
• **Bioiberica, S.A.U.**
  **08389 Palafolls Barcelona (ES)**
• **Universidad de Granada**
  **18071 Granada (ES)**

• **Universidad De Jaen**
  **23071 Jaen (ES)**

(72) Inventors:
• **Marchal Corrales, Juan Antonio**
  **Granada (ES)**
• **López Ruiz, Elena**
  **Jaén (ES)**
• **Gálvez Martín, Patricia**
  **Palafolls (ES)**
• **Chocarro Wrona, Carlos**
  **Granada (ES)**

(74) Representative: **Pons**
  **Glorieta Rubén Darío 4**
  **28010 Madrid (ES)**

(54) **HYDROGELS FOR USE IN SKIN TISSUE ENGINEERING**

(57)    The present invention relates to hydrogels comprising native skin components, as well as to bio-inks, compositions and dressings based on the same. Furthermore, the invention relates to their uses in regenerative medicine, in particular, in the regeneration, repair and replacement of skin tissue, and a method for obtaining the hydrogels of the invention.

(52) Cooperative Patent Classification (CPC): (Cont.)

C-Sets
**A61L 27/26, C08L 5/08;**
**A61L 27/26, C08L 5/12;**
**A61L 27/26, C08L 89/00;**
**A61L 27/26, C08L 89/06**

**Description**

The present invention belongs to the field of tissue engineering and biomedicine. In particular, the present invention relates to hydrogels comprising native skin components, as well as to bio-inks, compositions and dressings based on the same. Furthermore, the invention relates to their uses in regenerative medicine, in particular, in the regeneration, repair and replacement of skin tissue, and a method for obtaining the hydrogels of the invention.

**BACKGROUND OF THE INVENTION**

The skin is the largest organ in the human body, constituting a natural barrier against the external environment, which plays a fundamental role in preserving homeostasis and protecting the body's internal organs. Furthermore, it is also involved in many essential functions, such as the prevention of excessive transepidermal water loss, thermoregulation, sensory perception through specialised receptors or excretion.

That is why disorders or pathologies related to the skin affect the quality of life of the individuals who suffer from them. Disorders of this type may be caused, for example, by acute or chronic injuries, diabetic foot ulcers, perianal fistulas, epidermolysis bullosa, as well as traumas, burns or tears, among others.

A typical approach to the treatment of skin lesions involves replacing the lost tissue with an autograft, allograft or xenograft. Nonetheless, these solutions may have donor availability as a limitation.

Tissue engineering is a field that has provided many approaches aimed at solving disorders of this type. Tissue-engineered skin substitutes are an important field of research with a great impact on dermatopathology treatments. Skin substitutes must have biocompatible properties, help restore the epidermal barrier function and skin homeostasis (temperature, pH, TEWL, elasticity and moisture) and ensure a correct clinical result.

Various approaches of this type are described in the state of the art:

In the review by Dai C. *et al.* (Dai C. et al., Skin substitutes for acute and chronic wound healing: an updated review. J Dermatologist Treat., 31(6):639-648 (2020)), the advantages, drawbacks and indications of different skin substitutes and their clinical application are included and described.

Application CN106390205A discloses a 3D printed artificial skin and a method for preparing same, comprising, among others, a hydrogel layer containing keratinocytes, and a hydrogel layer containing hair follicle cells.

Application WO2019040224A1 discloses a hydrogel for tissue engineering and bioprinting. Specifically, it describes a cross-linked hydrogel composition that can be printed into a defined shape. The cross-linked hydrogel includes a plurality of biodegradable natural polymer macromers as well as the possibility of including cells.

Nevertheless, the biomaterials and skin models or substitutes comprising them that have been developed so far do not have ideal biomimicry, both in design and in composition, nor do they have physico-chemical and biological properties that limit their biomedical application.

That is why there is a need in the state of the art to develop alternative approaches based on biomaterials, with physico-chemical and biological properties suitable for their application as skin substitutes or models.

**DESCRIPTION OF THE INVENTION**

The present invention relates to hydrogels based on components of the skin, in particular, type I collagen, dermatan sulphate, elastin and hyaluronic acid, in addition to agarose, and which may further comprise live cells. Said hydrogels, furthermore, can have a monolaminar, bilaminar or trilaminar arrangement. The composition, organised in sheets or layers, as well as emerging properties of the hydrogels of the invention, allows them to biomimic the hypodermal, dermal and/or epidermal layers of the skin.

The inventors have shown that these hydrogels have physico-chemical properties that make them suitable for use in tissue engineering, for example, as bio-inks for 3D bioprinting (Fig. 1B, 1C, 1D and Fig. 2). In turn, said hydrogels or bio-inks comprising them were combined, giving rise to bilaminar or trilaminar skin models that showed biological properties giving them applicability in the regeneration, repair or replacement of skin (Fig. 4). In particular, *in vivo* assays have shown that the hydrogels of the invention, such as the trilaminar hydrogel or skin model (also herein referred to as BT Skin), effectively promote skin wound healing in an animal model of wound healing, having similar results and even surpassing the reference treatment or gold standard in some cases, as well as with respect to the control treatment (Fig. 5).

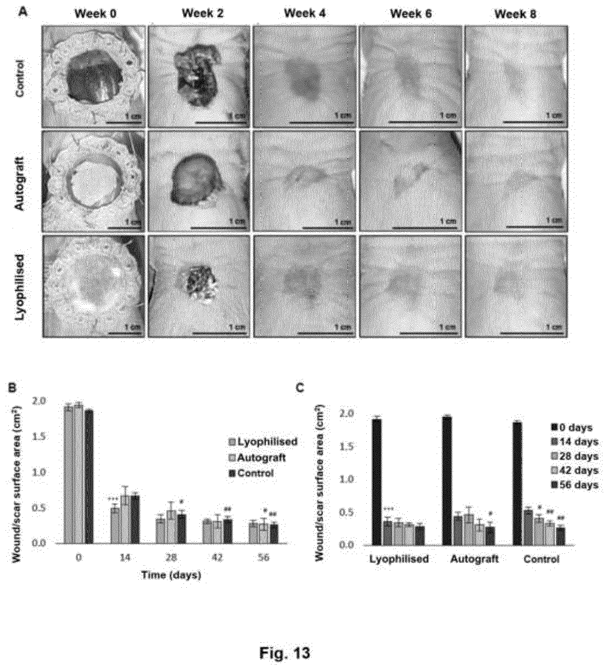

Likewise, some hydrogels of the invention demonstrated that they maintain their integrity after undergoing partial dehydration, which decreases their thickness and gives them improved mechanical properties for use in tissue engineering. Lastly, some hydrogels of the invention were lyophilised and characterised, exhibiting capacities to be applied in skin tissue engineering (Fig. 13).

The hydrogels of the invention have various advantages associated with their composition and arrangement, which emulate the different layers of the skin: they are biomimetic hydrogels, which potentially improves the response of a subject after its application in tissue regeneration; their physico-chemical properties, such as gel times and injectability, are optimal for use as bio-inks since they achieve good bioprintability; their biological properties, such as the maintenance of cell proliferation and high levels of cell viability, also give them the capacity for applications in regenerative medicine. All these mentioned advantages make the hydrogels of the invention, as well as bio-inks, compositions, or dressings comprising them, an alternative approach as skin models or substitutes, for their application in the field of tissue engineering and regenerative medicine.

On this basis, the inventors have developed a series of inventive aspects that will be described below:

Hydrogels of the invention and aspects derived therefrom

The inventors have shown that hydrogels comprising components present in the skin, in particular, type I collagen, elastin, dermatan sulphate and hyaluronic acid, in addition to agarose, and also possibly including other components, such as skin cells, have physico-chemical and biological properties of interest, especially for their application in tissue engineering.

The term "hydrogel", as used herein, refers to a network or mixture of materials, molecules, polymers or substances that are combined by chemical bonds, including covalent, ionic and supramolecular bonds, or by means of any combination thereof. The hydrogel may have a solid, semi-solid or semi-liquid texture, and can be used as a three-dimensional scaffold in tissue engineering.

Preferably, the hydrogels of the invention are biomimetic hydrogels. The term "biomimetic hydrogel", as used herein, refers to a hydrogel or scaffold that mimics the natural extracellular microenvironment to facilitate interaction between hydrogels and the surrounding cells through molecular recognition, and improves specific cell response and tissue regeneration.

Monolaminar, bilaminar and trilaminar hydrogels of the invention may herein be collectively referred to as "the hydrogels of the invention".

A hydrogel can be defined by components that comprise it, which in turn define its properties.

Type I collagen (Col I) is a protein that assembles into fibres that form the structural and mechanical scaffolding (matrix) of tissues, including skin, being an essential scaffolding protein that confers strength and elasticity to the same.

Dermatan sulphate (DS) is the main glucan in the extracellular matrix of the skin, participating in the reconstruction of said extracellular matrix in the wound healing process.

Hyaluronic acid is a polysaccharide that is also found in the dermal extracellular matrix, and which is widely known in the state of the art due to its utility in skin care products and tissue engineering, and given that it promotes wound healing and angiogenesis.

Agarose (Ag) is a polysaccharide present in certain algae, which has properties that make it useful for providing mechanical support and rapid gelification.

In the present invention, the hydrogels of the invention can be arranged or organised in single or multiple layers, in particular, in one layer (monolaminar hydrogel), two layers (bilaminar hydrogel), or three layers (trilaminar hydrogel), that mimic or biomimic the different main layers of the skin, the hypodermal layer, dermal layer and/or dermal layer.

*Monolaminar hydrogel*

One aspect of the invention relates to a monolaminar hydrogel, hereinafter "the monolaminar hydrogel of the invention", which comprises:

- Type I collagen (Col I),
- Dermatan sulphate (DS),
- Hyaluronic acid (HA), and
- Agarose (Ag).

Preferably, the monolaminar hydrogel further comprises elastin (EL).

The term "elastin" refers to a highly cross-linked protein, constituting a primary protein in the extracellular matrix of native skin, conferring elasticity to the dermal matrix.

In a preferred embodiment, the monolaminar hydrogel of the invention comprises Col I at a concentration from 1 to 3.5 mg/ml (including the end values), preferably 2.2 mg/ml; DS at a concentration from 7 to 10 mg/ml (including the end values), preferably 8.4 mg/ml; HA at a concentration from 0.5 to 1.5 mg/ml (including the end values), preferably 1 mg/ml; and Ag at a concentration from 10 to 30 mg/ml, preferably 15 mg/ml (including the end values). In a more preferred embodiment, the monolaminar hydrogel of the invention further comprises EL at a concentration from 0.5 to 1.5 mg/ml (including the end values), preferably 1 mg/ml.

In a preferred embodiment of the monolaminar hydrogel of the invention, alone or in combination with the other preferred embodiments, the hydrogel is lyophilised, hereinafter "the lyophilised monolaminar hydrogel of the invention". Preferably, the lyophilised monolaminar hydrogel of the invention does not comprise cells.

Furthermore, the hydrogels of the invention may comprise cells, preferably human cells.

Preferably, the cells of the hydrogels of the invention comprise cells that may be present in the different layers of the native skin. Examples of cell types that may be present in the skin include, but are not limited to, keratinocytes, melanocytes, Langerhans cells, Merkel cells, dermal fibroblasts, mastocytes, vascular smooth muscle cells, myoepithelial cells, histiocytes, neutrophils, lymphocytes (including B cells, T cells and regulatory T cells or Tregs), eosinophils, monocytes, stem cells, hair follicle stem cells, mesenchymal stem cells, adipocytes, NK cells (for its acronym Natural Killer).

In a preferred embodiment, alone or in combination with the other preferred embodiments, the monolaminar hydrogel of the invention comprises cells that may be present in the dermis and/or hypodermis of native skin. More preferably, the monolaminar hydrogel of the invention comprises cells selected from the list consisting of dermal fibroblasts, mastocytes, vascular smooth muscle cells, myoepithelial cells, histiocytes, neutrophils, lymphocytes (including B cells, T cells and regulatory T cells or Tregs), eosinophils, monocytes, stem cells, hair follicle stem cells, mesenchymal stem cells, adipocytes, NK cells, and any combination thereof. Even more preferably, the monolaminar hydrogel of the invention comprises mesenchymal stem cells and/or dermal fibroblasts.

The hydrogels of the invention can be organised, as already mentioned, in a single or multiple layers (or sheets). The term "layer", as used herein, refers to an association in the X, Y and Z planes of components of the extracellular matrix. Each layer can be defined by its composition and properties. Preferably, each layer has a homogeneous composition and/or properties therein.

The term "monolaminar", as used herein in relation to the hydrogel and its organisation or arrangement, refers to the fact that the components that make up the hydrogel are arranged in a layer.

*Bilaminar hydrogel*

Another aspect of the invention relates to a bilaminar hydrogel, hereinafter "the bilaminar hydrogel of the invention", comprising two layers of the monolaminar hydrogel of the invention. The bilaminar hydrogel of the invention comprises type I collagen, agarose, dermatan sulphate, hyaluronic acid and elastin.

As mentioned previously, the hydrogels of the invention can be organised in multiple layers (or sheets). Each layer can be defined by its associated composition and/or properties. Preferably, each layer has a homogeneous composition and/or properties therein, which differentiate them from each other. The layered organisation of the hydrogels of the invention allows emulating or mimicking the structure of body tissues, in particular, skin tissue, more particularly the main layers of the skin, providing them with a structural architecture that contributes to improving their biomimicry.

The term "bilaminar", as used herein in relation to the hydrogel and its organisation or arrangement, refers to the fact that the components that make up the hydrogel are arranged in two layers, a bottom layer and a top layer.

In a preferred embodiment of the bilaminar hydrogel of the invention, the bottom layer comprises type I collagen, agarose, dermatan sulphate and hyaluronic acid, and the top layer comprises type I collagen, agarose, dermatan sulphate, hyaluronic acid and elastin.

In another more preferred embodiment of the bilaminar hydrogel of the invention, both layers comprise elastin.

In another preferred embodiment, alone or in combination with the other preferred embodiments, the bilaminar hydrogel of the invention comprises Col I at a concentration from 1 to 3.5 mg/ml (including the end values), preferably 2.2 mg/ml; DS at a concentration from 7 to 10 mg/ml (including the end values), preferably 8.4 mg/ml; HA at a concentration from 0.5 to 1.5 mg/ml (including the end values), preferably 1 mg/ml; Ag at a concentration from 10 to 30 mg/ml (including the end values), preferably 15 mg/ml; and EL at a concentration from 0.5 to 1.5 mg/ml (including the end values), preferably 1 mg/ml.

In another more preferred embodiment of the bilaminar hydrogel of the invention:

-   the top layer comprises Col I at a concentration from 1 to 3.5 mg/ml (including the end values), DS at a concentration from 7 to 10 mg/ml (including the end values), HA at a concentration from 0.5 to 1.5 mg/ml (including the end values), Ag at a concentration from 10 to 30 mg/ml (including the end values), and EL at a concentration from 0.5 to 1.5 mg/ml (including the end values); and
-   the bottom layer comprises Col I at a concentration from 1 to 3.5 mg/ml (including the end values), DS at a concentration from 7 to 10 mg/ml (including the end values), HA at a concentration from 0.5 to 1.5 mg/ml (including the end values), Ag at a concentration from 10 to 30 mg/ml (including the end values), and EL at a concentration from 0.5 to 1.5 mg/ml (including the end values).

In another even more preferred embodiment of the bilaminar hydrogel of the invention:

- the top layer comprises Col I at a concentration of 2.2 mg/ml, DS at a concentration of 8.4 mg/ml, HA at a concentration of 1 mg/ml, Ag at a concentration of 15 mg/ml, and EL at a concentration of 1 mg/ml; and

- the bottom layer comprises Col I at a concentration of 2.2 mg/ml, DS at a concentration of 8.4 mg/ml, HA at a concentration of 1 mg/ml, Ag at a concentration of 15 mg/ml, and EL at a concentration of 1 mg/ml.

In a preferred embodiment of the bilaminar hydrogel of the invention, alone or in combination with the other preferred embodiments, the hydrogel is lyophilised, hereinafter "the lyophilised bilaminar hydrogel of the invention". Preferably, the lyophilised bilaminar hydrogel of the invention does not comprise cells.

Furthermore, the hydrogels of the invention may comprise cells, preferably human cells. Preferably, the cells of the hydrogels of the invention comprise cells that may be present in the different layers of the native skin. Examples of cell types that may be present in the skin include, but are not limited to, keratinocytes, melanocytes, Langerhans cells, Merkel cells, dermal fibroblasts, mastocytes, vascular smooth muscle cells, myoepithelial cells, histiocytes, neutrophils, lymphocytes (including B cells, T cells and regulatory T cells or Tregs), eosinophils, monocytes, stem cells, hair follicle stem cells, adipocytes, NK cells (for its acronym Natural Killer).

In a preferred embodiment, alone or in combination with the other preferred embodiments, the bilaminar hydrogel of the invention comprises cells that may be present in the dermis and/or hypodermis of native skin. More preferably, the bilaminar hydrogel of the invention comprises cells selected from the list consisting of dermal fibroblasts, mastocytes, vascular smooth muscle cells, myoepithelial cells, histiocytes, neutrophils, lymphocytes (including B cells, T cells and regulatory T cells or Tregs), eosinophils, monocytes, stem cells, hair follicle stem cells, adipocytes, NK cells, and any combination thereof. Even more preferably, the bilaminar hydrogel of the invention comprises mesenchymal stem cells and dermal fibroblasts.

The term "mesenchymal stem cells" (MSCs) refers herein to multipotent stromal cells that can differentiate into a variety of cell types, including osteoblasts (bone cells), chondrocytes (cartilage cells), myocytes (muscle cells), fibroblasts and adipocytes.

As understood by a person skilled in the art, methods for obtaining mesenchymal stem cells from a subject, preferably a mammal, more preferably a human, are widely known in the state of the art. Mesenchymal stem cells can be isolated from tissues or organs of a subject, for example and without being limited to, bone marrow, placenta, umbilical cord blood, Wharton's jelly, adipose tissue, adult muscle, corneal stroma, amniotic fluid, endometrium and dental tissue. Preferably, mesenchymal stem cells are isolated from adipose tissue.

The term "dermal fibroblasts" (DFs), as used in this document, refers to resident cells of connective tissue that synthesise fibres and maintain the extracellular matrix of tissues in many animals. In particular, dermal fibroblasts are found in the dermis layer of the skin, where they generate and maintain the connective tissue, playing a crucial role in wound healing and producing proteins for the extracellular matrix that connects the dermis and the epidermis.

In the present invention, the bilaminar hydrogel can mimic the structure and/or composition of the dermal and hypodermal layers of native skin.

Thus, in a preferred embodiment, alone or in combination with the other preferred embodiments, the bilaminar hydrogel of the invention comprises a first bottom layer comprising the monolaminar hydrogel of the invention, wherein said monolaminar hydrogel comprises mesenchymal stem cells, and a second top layer, arranged on top of the first, comprising the monolaminar hydrogel of the invention, wherein said monolaminar hydrogel comprises dermal fibroblasts. More preferably, the concentration of dermal fibroblasts in the top layer is 1M/ml, and the concentration of mesenchymal stem cells in the bottom layer is 1 M/ml.

In a preferred embodiment, alone or in combination with the other preferred embodiments, the dermal fibroblasts and/or mesenchymal stem cells are human.

Furthermore, other cells that may be comprised in the hydrogel include cells used in tissue regeneration, such as, but not limited to, primary cells, hair follicle stem cells, endothelial cells, nerve fibres, Pacinian corpuscles, Meissner's corpuscles, Krause's corpuscles, Ruffini's corpuscles.

Furthermore, the bilaminar hydrogel of the invention may have dimensions suitable for tissue engineering applications. In a preferred embodiment, alone or in combination with the other preferred embodiments, the bilaminar hydrogel of the invention comprises a width of 10 to 120 mm (including the end values), a length of 5 to 120 mm (including the end values) and a height of 0.2 to 10 mm (including the end values). More preferably, the bilaminar hydrogel of the invention comprises a width of 30 mm, a length of 15 mm and a height of 1.4 mm.

In certain embodiments of the bilaminar hydrogel of the invention, the thickness of the top layer is from 100 to 5000 $\mu$m. In certain embodiments, the thickness of the top layer is from 100 to 5000 $\mu$m. In certain embodiments, the thickness of the top layer is from 200 to 5000 $\mu$m. In certain embodiments, the thickness of the top layer is from 300 to 5000 $\mu$m. In certain embodiments, the thickness of the top layer is from 400 to 5000 $\mu$m. In certain embodiments, the thickness of the top layer is from 100 to 900 $\mu$m. In certain embodiments, the thickness of the top layer is from 100 to 800 $\mu$m. In certain embodiments, the thickness of the top layer is from 100 to 700 $\mu$m. In certain embodiments, the thickness of the top layer is from 100 to 600 $\mu$m. In certain embodiments, the thickness of the top layer is, at least, 100 $\mu$m. In certain embodiments, the thickness of the top layer is, at least, 200 $\mu$m. In certain embodiments, the thickness of the top layer is, at least, 300 $\mu$m. In certain embodiments, the thickness of the top layer is, at least, 400 $\mu$m. In certain embodiments, the thickness of the top layer is less than 2000 $\mu$m. In certain embodiments, the thickness of the top layer is less than 3000 $\mu$m. In certain embodiments, the thickness of the top layer is less than 4000 $\mu$m. In certain embodiments, the thickness of the top layer is less than 5000 $\mu$m. In certain embodiments, the thickness of the top layer is less than 1500 $\mu$m. In certain embodiments, the thickness of the top layer is less than 1000 $\mu$m. In certain embodiments, the thickness of the top layer is less than 900 $\mu$m. In certain embodiments, the thickness of the top layer is less than 800 $\mu$m. In certain embodiments, the thickness of the top layer is less than 700 $\mu$m. In certain embodiments, the thickness of the top layer is less than 600 $\mu$m. In certain embodiments, the thickness of the top layer is approximately 500 $\mu$m.

In certain embodiments of the bilaminar hydrogel of the invention, the thickness of the bottom layer is greater than 100 $\mu$m. In certain embodiments, the thickness of the bottom layer is greater than 200 $\mu$m. In certain embodiments, the thickness of the bottom layer is greater than 300 $\mu$m. In certain embodiments, the thickness of the bottom layer is greater than 400 $\mu$m. In certain embodiments, the thickness of the bottom layer is greater than 500 $\mu$m. In certain embodiments, the thickness of the bottom layer is greater than 1000 $\mu$m. In certain embodiments, the thickness of the bottom layer is greater than 2000 $\mu$m. In certain embodiments, the thickness of the bottom layer is greater than 3000 $\mu$m. In certain embodiments, the thickness of the bottom layer is greater than 4000 $\mu$m. In certain embodiments, the thickness of the bottom layer is greater than 5000 $\mu$m. In certain embodiments, the thickness of the bottom layer is greater than 1000 $\mu$m. In certain embodiments, the thickness of the bottom layer is less than about 500 $\mu$m. In specific embodiments, the thickness of the bottom layer is less than 400 $\mu$m. In specific embodiments, the thickness of the bottom layer is less than 300 $\mu$m. In certain embodiments, the thickness of the bottom layer is less than 200 $\mu$m. In certain embodiments, the thickness of the bottom layer is approximately 150 $\mu$m.

*Trilaminar hydrogel*

Another aspect of the invention relates to a trilaminar hydrogel, hereinafter "the trilaminar hydrogel of the invention", comprising:

- a bottom layer comprising the monolaminar hydrogel of the invention,
- a middle layer comprising the monolaminar hydrogel of the invention, and
- a top layer comprising a hydrogel comprising Col I, keratin (Kt), and sphingolipids (Sph).

As mentioned previously, the hydrogels of the invention can be organised in multiple layers (or sheets). Each layer can be defined by its composition and physico-chemical and/or biological properties. Preferably, each layer has a homogeneous composition and/or properties therein, which differentiate it. The layered organisation of the hydrogels of the invention allows them to emulate or mimic the structure of body tissues, providing them with a structural architecture that contributes to improving their biomimicry.

The term "trilaminar", as used herein in relation to the hydrogel and its organisation or arrangement, refers to the fact that the components that make up the hydrogel are arranged in three layers.

In the present invention, the trilaminar hydrogel mimics the structure of the skin. The skin has three differentiated layers, arranged from most superficial to least as indicated below: the epidermis or epidermal layer, the dermis or dermal layer, and the hypodermis or hypodermal layer. The "hypodermal layer" of the skin is a layer located directly below the dermis. This layer is made up of well-vascularised areolar or loose connective tissue and adipose tissue, which functions as a mode of fat storage and provides insulation and cushioning. The "dermal layer" of the skin is the middle layer of the skin. This layer has connective tissue and other structures. It is made up of a thin top layer called the papillary dermis and a thick bottom layer called the reticular dermis. The "epidermal layer" of the skin is a thin layer that constitutes the outer surface of the skin. This layer, in turn, can comprise several layers or strata, called stratum corneum, stratum lucidum, stratum spinosum, stratum granulosum and stratum basale.

Thus, in the present invention, the trilaminar hydrogel is organised in three layers, each of which in turn comprises a hydrogel (or bio-ink comprising it):

The bottom layer of the trilaminar hydrogel comprises the monolaminar hydrogel of the invention, comprising type I collagen (Col I), dermatan sulphate (DS), hyaluronic acid (HA), and agarose (Ag). Preferably, said bottom layer, arranged under the middle layer, biomimics the hypodermal layer of the skin.

The middle layer of the trilaminar hydrogel comprises the monolaminar hydrogel of the invention, comprising type I collagen (Col I), dermatan sulphate (DS), hyaluronic acid (HA) and agarose (Ag). Preferably, said middle layer of the trilaminar hydrogel further comprises elastin. More preferably, said middle layer biomimics the dermal layer of the skin.

The top layer of the trilaminar hydrogel comprises Col I, keratin (Kt) and sphingolipids (Sph). Preferably, said top layer, arranged on top of the middle layer of the trilaminar hydrogel, biomimics the epidermal layer of the skin.

In a preferred embodiment of the trilaminar hydrogel of the invention:

- the top layer comprises Col I at a concentration from 3.5 to 5.5 mg/ml (including the end values), Kt at a concentration from 10 to 30 mg/ml (including the end values) and Sph at a concentration from 2.5 to 7.5 mg/ml (including the end values);

- the middle layer comprises Col I at a concentration from 1 to 3.5 mg/ml (including the end values), DS at a concentration from 7 to 10 mg/ml (including the end values), HA at a concentration from 0.5 to 1.5 mg/ml (including the end values), Ag at a concentration from 10 to 30 mg/ml (including the end values), and EL at a concentration from 0.5 to 1.5 mg/ml (including the end values); and

- the bottom layer comprises Col I at a concentration from 1 to 3.5 mg/ml (including the end values), DS at a concentration from 7 to 10 mg/ml (including the end values), HA at a concentration from 0.5 to 1.5 mg/ml (including the end values), and Ag at a concentration from 10 to 30 mg/ml (including the end values).

In another more preferred embodiment of the trilaminar hydrogel of the invention:

- the top layer comprises Col I at a concentration of 4.4 mg/ml, Kt at a concentration of 15.2 mg/ml and Sph at a concentration of 5 mg/ml;

- the middle layer comprises Col I at a concentration of 2.2 mg/ml, DS at a concentration of 8.4 mg/ml, HA at a concentration of 1 mg/ml, Ag at a concentration of 15 mg/ml, and EL at a concentration of 1 mg/ml; and

- the bottom layer comprises Col I at a concentration of 2.2 mg/ml, DS at a concentration of 8.4 mg/ml, HA at a concentration of 1 mg/ml, and Ag at a concentration of 15 mg/ml.

In a preferred embodiment of the trilaminar hydrogel of the invention, alone or in combination with the other preferred embodiments, the hydrogel is lyophilised, hereinafter "the lyophilised trilaminar hydrogel of the invention". Preferably, the lyophilised trilaminar hydrogel of the invention does not comprise cells.

In the present invention, the term "lyophilised" refers to the fact that the product in the present invention, the trilaminar, monolaminar or bilaminar hydrogel of the invention, as well as the dressing of the invention described below, was subjected to a lyophilisation process. The term "lyophilisation" refers to the rapid freezing and dehydration of the product under high vacuum conditions. In the present invention, the term "lyophilised hydrogels of the invention" is used to refer collectively to the lyophilised monolaminar hydrogel of the invention, lyophilised bilaminar hydrogel of the invention and the lyophilised trilaminar hydrogel of the invention.

In a preferred embodiment, alone or in combination with the other preferred embodiments, the monolaminar, bilaminar or trilaminar hydrogel of the invention is dehydrated.

Furthermore, as mentioned previously, the hydrogels of the invention may comprise cells, preferably human cells. Preferably, the cells of the hydrogels of the invention comprise cells that may be present in the different layers of the native skin. Examples of cell types that may be present in the skin include, but are not limited to, keratinocytes, melanocytes, Langerhans cells, Merkel cells, dermal fibroblasts, mastocytes, vascular smooth muscle cells, myoepithelial cells, histiocytes, neutrophils, lymphocytes (including B cells, T cells and regulatory T cells or Tregs), eosinophils, monocytes, stem cells, hair follicle stem cells, mesenchymal stem cells, adipocytes, NK cells (for its acronym Natural Killer).

Thus, in a preferred embodiment, alone or in combination with the other preferred embodiments, the trilaminar hydrogel of the invention further comprises cells selected from the list consisting of keratinocytes, melanocytes, Langerhans cells, Merkel cells, dermal fibroblasts, mastocytes, vascular smooth muscle cells, myoepithelial cells, histiocytes, neutrophils, lymphocytes (including B cells, T cells and regulatory T cells or Tregs), eosinophils, monocytes, mesenchymal stem cells, adipocytes, NK cells, and any combination thereof. Preferably, the trilaminar hydrogel of the invention comprises dermal fibroblasts (DFs), mesenchymal stem cells (MSCs) and/or epidermal keratinocytes (EKs). More preferably, the top layer comprises EKs, the middle layer comprises DFs and the bottom layer comprises MSCs. Even more preferably, the DFs, MSCs and/or EKs are human.

The terms "mesenchymal stem cells" and "dermal fibroblasts" have been previously explained in another aspect of the invention, and both they and their preferred embodiments are applicable to the present aspect of the invention.

The term "epidermal keratinocytes" (EK), as used herein, refers to the main cell type of the epidermis, constituting approximately 90% of its cells. Epidermal keratinocytes proliferate in the stratum basale of the epidermis and begin to differentiate on their way to the surface, undergoing gradual differentiation. During this process, they profoundly change their morphology and begin to produce keratin, cytokines, growth factors, interleukins and complement factors. Keratinocytes play an important role in protection, since they form a hermetic barrier that prevents the entry of foreign substances into the body, while minimising loss of moisture, heat and other components. These cells also have a structural role, forming close bonds with the other cells of the epidermis and holding them in place. Furthermore, keratinocytes function as immunomodulators after skin lesions.

In a preferred embodiment, alone or in combination with the other preferred embodiments, mesenchymal stem cells come from adipose tissue, preferably human adipose tissue. In another preferred embodiment, alone or in combination with the other preferred embodiments, dermal fibroblasts and/or epidermal keratinocytes come from skin samples, preferably human.

In another preferred embodiment, alone or in combination with the other preferred embodiments, the concentration of MSCs in the bottom layer of the trilaminar hydrogel of the invention is 1 M/ml and the concentration of DFs in the middle layer of the trilaminar hydrogel of the invention is 1 M/ml.

In another preferred embodiment, alone or in combination with the other preferred embodiments, the concentration of EKs in the trilaminar hydrogel of the invention is 1M/cm$^2$.

Furthermore, other cells that may be comprised in the trilaminar hydrogel of the invention include cells used in tissue regeneration, such as, but not limited to, hair follicle stem cells, endothelial cells, nerve fibres, Pacinian corpuscles, Meissner's corpuscles, Krause's corpuscles, Ruffini's corpuscles.

In a preferred embodiment, the trilaminar hydrogel of the invention is dehydrated. Dehydration methods are widely known in the state of the art. Preferably, the dehydrated trilaminar hydrogel is obtained by a dehydration step that comprises applying pressure, more preferably, applying 50 to 150 g of pressure, including the end values. Even more preferably, dehydration comprises applying 50, 55, 60, 65, 70, 75, 80, 85, 90, 95, 100, 105, 110, 115, 120, 125, 130, 135, 140, 145 or 150 g of pressure.

In another preferred embodiment, the dehydration comprises applying pressure for 1 to 5 minutes. Preferably, it comprises applying pressure for 1, 2, 3, 4, or 5 minutes. More preferably, it comprises applying pressure for 2 minutes.

In another more preferred embodiment, the dehydration comprises applying 100 g of pressure for 2 minutes.

Furthermore, the trilaminar hydrogel of the invention may have dimensions suitable for tissue engineering applications. In a preferred embodiment, alone or in combination with the other preferred embodiments, the trilaminar hydrogel of the invention comprises a diameter of 10 to 40 mm (including the end values), and a height of 0.3 to 10 mm (including the end values). More preferably, the bilaminar hydrogel of the invention comprises a diameter of 20 mm, and a height of 4 mm.

In certain embodiments of the trilaminar hydrogel of the invention, the thickness of the top layer is from 100 to 500 μm. In certain embodiments, the thickness of the top layer is from 100 to 400 μm. In certain embodiments, the thickness of the top layer is from 100 to 300 μm. In certain embodiments, the thickness of the top layer is from 100 to 200 μm. In certain embodiments, the thickness of the top layer is from 100 to 400 μm. In specific embodiments, the thickness of the top layer is greater than about 300 μm. In specific embodiments, the thickness of the top layer is greater than 100 μm. In specific embodiments, the thickness of the top layer is greater than 200 μm. In specific embodiments, the thickness of the top layer is greater than 300 μm. In specific embodiments, the thickness of the top layer is greater than 400 μm. In specific embodiments, the thickness of the top layer is greater than 500 μm. In specific embodiments, the thickness of the top layer is greater than 600 μm. In specific embodiments, the thickness of the top layer is less than about 500 μm. In specific embodiments, the thickness of the top layer is less than 400 μm. In specific embodiments, the thickness of the top layer is less than 300 μm. In specific embodiments, the thickness of the top layer is less than 200 μm. In certain embodiments, the thickness of the top layer is approximately 150 μm.

In certain embodiments of the trilaminar hydrogel of the invention, the thickness of the middle layer is from 100 to 1000 μm. In certain embodiments, the thickness of the middle layer is from 200 to 1000 μm. In certain embodiments, the thickness of the middle layer is from 300 to 1000 μm. In certain embodiments, the thickness of the middle layer is from 400 to 1000 μm. In certain embodiments, the thickness of the middle layer is from 100 to 900 μm. In certain embodiments, the thickness of the middle layer is from 100 to 800 μm. In certain embodiments, the thickness of the middle layer is from 100 to 700 μm. In certain embodiments, the thickness of the middle layer is from 100 to 600 μm. In certain embodiments, the thickness of the middle layer is, at least, 100 μm. In certain embodiments, the thickness of the middle layer is, at least, 200 μm. In certain embodiments, the thickness of the middle layer is, at least, 300 μm. In certain embodiments, the thickness of the middle layer is, at least, 400 μm. In certain embodiments, the thickness of the middle layer is less than 2000 μm. In certain embodiments, the thickness of the middle layer is less than 1500 μm. In certain embodiments, the thickness of the middle layer is less than 1000 μm. In certain embodiments, the thickness of the middle layer is less than 900 μm. In certain embodiments, the thickness of the middle layer is less than 800 μm. In certain embodiments, the thickness of the middle layer is less than 700 μm. In certain embodiments, the thickness of the middle layer is less than 600 μm. In certain embodiments, the thickness of the middle layer is approximately 500 μm.

In certain embodiments of the trilaminar hydrogel of the invention, the thickness of the bottom layer is greater than about 100 μm. In certain embodiments, the thickness of the bottom layer is greater than 200 μm. In certain embodiments, the thickness of the bottom layer is greater than 300 μm. In certain embodiments, the thickness of the bottom layer is greater than 400 $\mu$m. In certain embodiments, the thickness of the bottom layer is greater than 500 $\mu$m. In certain embodiments, the thickness of the bottom layer is greater than 600 $\mu$m. In certain embodiments, the thickness of the bottom layer is greater than 700 $\mu$m. In certain embodiments, the thickness of the bottom layer is greater than 800 $\mu$m. In certain embodiments, the thickness of the bottom layer is greater than 900 $\mu$m. In certain embodiments, the thickness of the bottom layer is greater than 1000 $\mu$m. In certain embodiments, the thickness of the bottom layer is greater than 200 $\mu$m. In certain embodiments, the thickness of the bottom layer is greater than 300 $\mu$m. In certain embodiments, the thickness of the bottom layer is greater than 400 $\mu$m. In certain embodiments, the thickness of the bottom layer is greater than 500 $\mu$m. In certain embodiments, the thickness of the bottom layer is greater than 125 $\mu$m. In certain embodiments, the thickness of the bottom layer is less than about 500 $\mu$m. In specific embodiments, the thickness of the bottom layer is less than 400 $\mu$m. In specific embodiments, the thickness of the bottom layer is less than 300 $\mu$m. In certain embodiments, the thickness of the bottom layer is less than 200 $\mu$m. In certain embodiments, the thickness of the bottom layer is approximately 150 $\mu$m.

*Bio-inks of the invention*

The inventors have characterised bio-inks based on the hydrogels of the invention, demonstrating that they have suitable physico-chemical properties for their practical application, such as their pH, injectability, gel time, degradation or rheology.

Thus, another aspect of the invention relates to a bio-ink comprising any of the hydrogels of the invention.

Another aspect of the invention relates to the use of any of the hydrogels of the invention for the manufacture of a bio-ink.

The term "bio-ink", as used herein, refers to a hydrogel or a mixture of components in the present invention, components that may be present in native skin, possibly including cells, used for bioprinting and/or spraying. The texture of the bio-ink can be liquid, semi-solid, or solid.

The term "bioprinting", or more particularly "3D bioprinting", refers to the use of printing techniques, preferably 3D printing, to combine biomaterials in order to manufacture an object, part, tissue or 3D structure that closely mimics natural and biological characteristics. Preferably, 3D bioprinting uses the layer-by-layer method to deposit materials or bio-inks to create structures that are later used in fields of biotechnology, medicine and tissue engineering.

As is known to a person skilled in the art, the bio-ink may further comprise other useful components in the bioprinting process or in the final product. Examples of such components include, but are not limited to, alginate, gelatin from various sources (skin from fish, bovine animals, swine), fibrinogen from various sources (plasma from bovine animals, swine, humans), intra-articular sodium hyaluronate, silk fibroin and sericin, xanthan, genipin, agarose, agar, chitosan, nanocellulose, or their methacrylated variants, or any combination thereof.

Different bio-inks can be differentiated depending on the hydrogel of the invention on which they are based. In the present document, the term "bio-inks of the invention" is used to refer to bio-inks collectively, or to any of the bio-inks of the invention.

Another aspect of the invention relates to the use of the monolaminar hydrogel as a hypodermal or dermal bio-ink.

A preferred embodiment relates to the use of the monolaminar hydrogel as a hypodermal bio-ink when the hydrogel comprises type I collagen, agarose, dermatan sulphate and hyaluronic acid. Another more preferred embodiment relates to the use of the monolaminar hydrogel as a hypodermal bio-ink, when the hydrogel comprises type I collagen, agarose, dermatan sulphate, hyaluronic acid and mesenchymal stem cells, preferably wherein the mesenchymal stem cells are human.

Another preferred embodiment relates to the use of the monolaminar hydrogel as a dermal bio-ink when the hydrogel comprises type I collagen, agarose, dermatan sulphate, hyaluronic acid and elastin. Another more preferred embodiment relates to the use of the monolaminar hydrogel as a dermal bio-ink when the hydrogel comprises type I collagen, agarose, dermatan sulphate, hyaluronic acid, elastin and dermal fibroblasts, preferably wherein the dermal fibroblasts are human.

Another aspect of the invention relates to the use of a hydrogel comprising Col I, keratin and sphingolipids as an epidermal bio-ink. A preferred embodiment relates to the use of a hydrogel comprising Col I, keratin, sphingolipids and keratinocytes, as an epidermal bio-ink, preferably wherein the keratinocytes are human.

The terms explained for the bio-inks of the invention have been previously explained in other aspects of the invention, and both they and their preferred embodiments are applicable to the bio-inks of the invention.

*Dressing of the invention*

The hydrogels of the invention, including the lyophilised hydrogels of the invention, and bio-inks of the invention, can be part of a dressing or implant. In fact, the hydrogels and bio-inks of the invention can be used for the manufacture of a dressing or implant.

Thus, another aspect of the present invention relates to the use of a hydrogel or hydrogels of the invention, or a bio-ink or bio-inks of the invention for the manufacture of a dressing or implant, preferably cutaneous or subcutaneous.

Another aspect of the invention relates to a dressing or implant, hereinafter "the dressing or implant of the invention", preferably cutaneous or subcutaneous, which comprises a hydrogel or hydrogels of the invention, or a bio-ink or bio-inks of the invention.

The terms "dressing" or "implant" are used synonymously and refer to any object or element that is biocompatible with the physiological state of the subject's body and does not produce adverse side effects, and that is structured, designed or configured to be placed partially or totally on a subject's body for one or more therapeutic or prophylactic purposes, such as increasing the tissues, the contour, restoring physiological function, repairing or restoring tissue damaged by disease or trauma, and/or administering therapeutic agents to normal, damaged or sick organs and tissue.

In a preferred embodiment, the dressing or implant is lyophilised. The term "lyophilised" has already been explained in other aspects of the invention, and both its definition and preferred embodiments are applicable to the present aspect of the invention.

The dressing or implant can be solid, semi-solid or liquid, and can be amorphous, spherical, hemispherical, rectangular, square, discoidal or cylindrical. Furthermore, for example, the dressing diameter can be from 0.05 mm to 100 mm, from 0.1 mm to 50 mm, from 0.1 mm to 30 mm, or from 0.2 mm to 15 mm, and can be provided in such size or shape. Furthermore, the dressing or implant may be applied to a target site (for example, a site of tissue damage), implanted or it can change its shape based on the shape of the damaged site.

The terms of the present aspect of the invention have been previously explained in other aspects of the invention, and both they and their preferred embodiments are applicable to the dressing of the invention.

*Pharmaceutical composition of the invention*

The monolaminar, bilaminar or trilaminar hydrogel of the invention, as well as the bio-ink(s) of the invention, may be comprised in a composition, hereinafter "composition of the invention". Thus, in another aspect, the invention relates to a composition, preferably a pharmaceutical composition, hereinafter "the pharmaceutical composition of the invention", comprising the monolaminar hydrogel, the bilaminar hydrogel, the trilaminar hydrogel of the invention or the bio-ink(s) of the invention.

The compositions of the present invention may be formulated for administration to an animal, more preferably to a mammal, including a human, in a wide variety of forms known in the state of the art. In that sense, they can be in, but are not limited to, aqueous or non-aqueous solutions, emulsions or suspensions. Examples of non-aqueous solutions are, for example, but not limited to, propylene glycol, polyethylene glycol, vegetable oils, such as olive oil, or injectable organic esters, such as ethyl oleate. Examples of aqueous solutions are for example, but not limited to, water, alcoholic solutions in water, or saline media. Aqueous solutions may or may not be buffered, and may have additional active or inactive components. Additional components include salts to modulate ionic strength, preservatives including, but not limited to, antimicrobial agents, antioxidants, chelating agents, or the like, or nutrients, including glucose, dextrose, vitamins and minerals. Alternatively, the compositions can be prepared for administration in solid form. The compositions can be combined with several inert carriers or excipients, including but not limited to: binders, such as microcrystalline cellulose, gum tragacanth, or gelatin; excipients, such as starch or lactose; dispersing agents, such as alginic acid or corn starch; lubricants, such as magnesium stearate, glidants such as colloidal silicon dioxide; sweetening agents, such as sucrose or saccharin; or flavouring agents, such as peppermint or methyl salicylate.

As understood by a person skilled in the art, the composition according to the present invention can be formulated with an excipient and/or a carrier. In that sense, in a particular embodiment, the composition of the invention comprises an excipient and/or a carrier. In the case of the pharmaceutical composition, it can be formulated with a pharmaceutically acceptable excipient and/or carrier.

The term "excipient" refers to a substance which helps absorb some of the components of the composition of the invention, stabilises said components or helps in the preparation of the composition, such as giving it consistency. In that sense, the excipients may have the function of holding the components together, such as starches, sugars or celluloses, a sweetening function, a colouring function, the function of protecting the medicine, such as isolating it from air and/or moisture, a function of filling a tablet, a capsule or any other formulation form, such as dibasic calcium phosphate, the function of disintegrating in order to facilitate the dissolution of components and their absorption in the intestine, without excluding other types of excipients not mentioned in this paragraph. Thus, the term "excipient" is defined as any material included in the galenic forms that is added to the active ingredients or to their associations to allow their preparation and stability, to modify their organoleptic properties or to determine the physico-chemical properties of the composition and its bioavailability. The "pharmaceutically acceptable" excipient must allow for the activity of the compounds of the pharmaceutical composition, in other words, it is compatible with said components. Examples of excipients are binders, weightings, disintegrators, lubricants, coatings, sweeteners, flavourings and dyes. Non-limiting and more specific examples of acceptable excipients are starches, sugars, xylitol, sorbitol, calcium phosphate, steroid fats, talc, silica or glycerin, among others.

The term "carrier" refers to a compound that facilitates the incorporation of other compounds to allow better dosage and administration or to give consistency and shape to the composition. Therefore, the carrier is a substance that is used to dilute some of the components of the composition of the present invention to a certain volume or weight, or even without diluting said components, being able to allow better dosage and administration or give consistency. When the form of presentation is liquid, the carrier is the diluent. In a particular embodiment, the carrier is pharmaceutically acceptable.

In a preferred embodiment, the pharmaceutical composition of the invention further comprises at least one active ingredient able to modulate the properties/functions of the tissue or areas in which it is applied or administered.

In a preferred embodiment, the pharmaceutical composition of the invention further comprises at least one active ingredient that favours the repair or regeneration of skin tissue.

The active ingredient can include polynucleotides and/or polypeptides that encode or comprise, for example, transcription factors, differentiating factors, growth factors and combinations thereof. The phrase "at least one active agent" can also include any agent able to promote tissue formation (for example, skin tissue), and/or target a specific disease state, preferably disease that affects the skin. Preferably, the active ingredient is a bioactive agent. Examples of bioactive agents include, but are not limited to, chemotactic agents, glycoproteins, lipoproteins, cell adhesion mediators, biologically active ligands, integrin-binding sequence, various growth and/or differentiation agents and fragments thereof (for example, EGF), HGF, VEGF, fibroblast growth factors (for example, bFGF), PDGF, insulin-like growth factor (for example, IGF-I, IGF-II) and transforming growth factors (for example, TGF-$\beta$ I-III), growth differentiation factors (for example, GDF5, GDF6, GDF8), recombinant human growth factors (for example, MP-52 and the MP-52 rhGDF-5 variant), cartilage-derived morphogenic proteins (CDMP-1, CDMP-2, CDMP-3), small molecules that affect the regulation of specific growth factors, tenascin-C, chondroitin sulphate, fibronectin, decorin, thromboelastin; thrombin-derived peptides, heparin-binding domains, heparin, heparin sulphate, polynucleotides, DNA fragments, DNA plasmids, MMP, TIMP, interfering RNA Molecules, DNA encoding an RNA of interest, oligonucleotides, proteoglycans, glycoproteins and glycosaminoglycans.

The route of administration of the composition of the invention, preferably the pharmaceutical composition of the invention, and/or the formulations thereof, as well as the hydrogels of the invention, hydrogels obtained by the obtaining method of the invention, the bio-inks of the invention, includes, but is not limited to, parenteral and intravenous administration, intraperitoneal, intramuscular, or intra-articular injection, dermal administration, intradermal administration, topical administration, intranasal administration, oral administration, or transmucosal administration.

In a preferred embodiment, the route of administration is selected from the list consisting of topical, dermal, intradermal, subcutaneous and intralesional administration.

Uses of the invention

The hydrogels of the invention, as well as the aspects derived therefrom, have several uses and applications, which will be described below.

*Therapeutic uses*

As previously explained, the inventors have shown that the hydrogels of the invention and aspects derived therefrom have physico-chemical and biological properties of interest, especially for their application in tissue engineering, in addition to being able to promote wound healing, being applicable in medicine, more particularly in regenerative medicine and/or in the regeneration, repair, or replacement of a subject's skin tissue.

In the present invention, "subject" is understood to be any animal, preferably a mammal, more preferably a primate, in particular, a human being, of any race, sex or age.

Thus, another aspect of the invention relates to one of the hydrogels of the invention, hydrogels of the invention, hydrogels obtained by the obtaining method of the invention, bio-inks of the invention, or pharmaceutical composition of the invention, for use in medicine, preferably in regenerative medicine.

In another aspect, the invention relates to one of the hydrogels of the invention, the hydrogels of the invention, hydrogels obtained by the obtaining method of the invention, or bio-ink of the invention for use in medicine, preferably in regenerative medicine, wherein the hydrogel, hydrogels, hydrogels obtained by the obtaining method, or bio-ink of the invention is provided in the form of an implant.

Another aspect of the invention relates to one of the hydrogels of the invention, hydrogels of the invention, hydrogels obtained by the obtaining method of the invention, bio-inks of the invention, or pharmaceutical composition of the invention, for use in the regeneration, repair or replacement of skin tissue.

Skin tissue includes tissue of the epidermal, dermal and hypodermal layers. Thus, a preferred embodiment relates to the hydrogels of the invention (monolaminar hydrogel, bilaminar hydrogel and/or trilaminar hydrogel), the bio-ink of the invention or the pharmaceutical composition of the invention for use in the regeneration, repair and/or replacement of the epidermis, dermis and/or hypodermis.

The term "regeneration", as used herein, refers to the complete replacement of damaged tissue with new tissue.

The term "repair", as used herein, refers to the restoration of a tissue structure that has previously suffered damage, as well as the restoration of its components and/or function.

The term "replacement", as used herein, refers to a substitution or reproduction of properties present in native tissue.

Another aspect of the invention relates to one of the hydrogels of the invention, hydrogels of the invention, hydrogels obtained by the obtaining method of the invention, bio-inks of the invention, or pharmaceutical composition of the invention for use in the treatment of skin lesions or wounds.

As used herein, the term "treatment" (interchangeable term with "therapy" and which can be used interchangeably herein) refers to processes that involve a slowdown, interruption, stoppage, control, reduction or reversal of the progression or severity of an existing symptom, disorder, condition or disease, but it does not necessarily imply complete elimination of all symptoms, conditions or disorders related to the disease. Furthermore, "treatment" can also refer to a process that involves preventing the onset of symptoms that have not yet manifested, but will manifest as a result of untreated progression of the disorder, condition or disease. The treatment of a disorder or disease may, for example, lead to stopping the progression of the disorder or disease (for example, without deterioration of symptoms) or to a delay in the progression of the disorder or disease (if the stoppage of progression is only temporary). The "treatment" of a disorder or disease can also lead to a partial response (for example, improvement of symptoms) or complete response (for example, the disappearance of symptoms) of the subject/patient suffering from the disorder or disease. Accordingly, the "treatment" of a disorder or disease can also refer to an improvement of the disorder or disease, which can, for example, lead to stopping the progression of the disorder or disease or to a delay in the progression of the disorder or disease. Said partial or complete response may be followed by a relapse. It is to be understood that a subject/patient may experience a wide range of responses to a treatment (such as the exemplary responses described above). In the present invention, the condition or disorder to be treated are illnesses or pathologies that affect the skin, preferably skin lesions or wounds.

*Uses of the hydrogels of the invention, and aspects derived therefrom, in the manufacture of a medicament.*

As already mentioned, the hydrogels of the invention and aspects derived therefrom are applicable in medicine, more particularly in regenerative medicine, and/or in the regeneration, repair, or replacement of a subject's skin tissue.

Thus, in another aspect, the invention relates to the use of one of the hydrogels of the invention, hydrogels of the invention, hydrogels obtained by the obtaining method of the invention, or bio-inks of the invention, in the preparation of a pharmaceutical composition or medicament. The techniques and methods for preparing pharmaceutical compositions are known in the state of the art. In turn, the term pharmaceutical composition, as well as its preferred embodiments have already been explained herein.

In another aspect, the invention relates to the use of one of the hydrogels of the invention, hydrogels of the invention, hydrogels obtained by the obtaining method of the invention, or bio-ink of the invention, in the preparation of a pharmaceutical composition to regenerate, repair or replace skin tissue.

In another aspect, the invention relates to the use of one of the hydrogels of the invention, hydrogels of the invention, hydrogels obtained by obtaining method of the invention, or bio-inks of the invention, in the preparation of a pharmaceutical composition for the treatment of skin lesions or wounds.

The terms used to define said aspects of the invention have already been explained previously, and both they and their preferred embodiments are applicable thereto.

*Cosmetic use*

Another aspect of the invention relates to a cosmetic composition, hereinafter "the cosmetic composition of the invention" comprising the monolaminar hydrogel, the bilaminar hydrogel, the trilaminar hydrogel of the invention or the bio-ink(s) of the invention. The cosmetic composition of the invention can be prepared in various ways, for example, emulsions, lotions, creams (oil in water, water in oil, multiphase), solutions, suspensions, anhydrous products (oil and glycol-based), gels, masks, packages, powders and the like. Furthermore, the cosmetic composition of the present invention may include an acceptable carrier in a cosmetic preparation. Here, the "acceptable carrier in the cosmetic preparation" is a compound known in the state of the art that can be included in the cosmetic preparations.

Examples of acceptable carriers in the cosmetic preparation include, but are not limited to, alcohols, oils, surfactants, fatty acids, silicone oils, wetting agents, moisturising agents, viscosity modifiers, emulsions, stabilisers, sunscreens, dyes, fragrances and the like.

Another aspect of the invention relates to the cosmetic use of any of the hydrogels of the invention, bio-ink of the invention, composition of the invention, preferably the cosmetic composition of the invention, or dressing of the invention.

The term "cosmetic use" refers to a non-therapeutic use that can improve the aesthetic appearance or comfort of some area, organ or tissue of a subject in the present invention related to skin, as well as its improvement and/or prevention of damage. Preferably, the subject is a human.

*In vitro uses of the hydrogels of the invention, and aspects derived therefrom*

In addition to the aforementioned applications, it is also possible to apply the hydrogel, as well as the inventive aspects derived therefrom in *in vitro* assays.

Thus, another aspect of the invention refers to the use of one of the hydrogels of the invention, hydrogels of the invention, hydrogels obtained by the obtaining method of the invention, bio-ink of the invention, composition of the invention, or dressing of the invention to promote *in vitro* wound healing.

The term "healing", as used herein, refers to a natural process that the body has to regenerate compromised tissues, skin tissues in the present invention, due to a wound. In this process, a series of complex biochemical phenomena are carried out, taking place to repair the damage caused by the wound.

The term "wound", as used herein, refers to a disorder in which areas or tissues are cut, torn, burned or traumatised, causing damage. It can also refer to a lesion or disorder in the human body caused by a disease. Furthermore, the wound may be a wound caused by another disease. For example, the wound may be fibrosis, diabetes, a diabetic ulcer, an autoimmune skin disease, an abrasion, a laceration, a cut, a bruise, a prick, shedding, a burn, an ulcer, a bedsore or a combination thereof.

In a preferred embodiment, the wound is selected from the group consisting of a chronic wound, acute wound, surgical wound, orthopaedic wound, traumatic wound, combat wound, and any combination thereof.

Furthermore, the hydrogels of the invention and aspects derived therefrom have shown the ability to restore melanin levels. Thus, another aspect of the invention relates to the use of one of the hydrogels of the invention, the hydrogels of the invention, hydrogels obtained by the obtaining method of the invention, bio-inks of the invention, composition or dressing of the invention to promote *in vitro* melanin production in isolated skin cells. Preferably, the isolated cells are from human skin.

The hydrogels of the invention, the dressing of the invention or the bio-inks of the invention can be used as a skin model, including a pathology model for skin, lesions or tumours. Thus, another aspect of the invention relates to the *in vitro* use of one of the hydrogels of the invention, the hydrogels of the invention, hydrogels obtained by the obtaining method the invention, bio-ink of the invention, or dressing of the invention as an artificial skin model. Preferably, it relates to the *in vitro* use as an artificial skin model in laboratory tests or assays to evaluate the effects exerted by substances under study.

Furthermore, the hydrogels of the invention, the dressing of the invention or the bio-inks of the invention can be used to test drugs and/or compounds toxic to the skin.

Thus, another aspect of the invention relates to the *in vitro* use of one of the hydrogels of the invention, the hydrogels of the invention, hydrogels obtained by the obtaining method of the invention, bio-ink of the invention, or dressing of the invention to test drugs and/or compounds toxic to the skin.

As used herein, the term "testing" refers to measuring or assessing the potential effect on the skin of substances under study, such as chemical compounds, molecules, toxic compounds, or drugs. It also refers to assessing the effect of substances under study, to identify drugs and/or compounds toxic to the skin.

Method for obtaining the hydrogel

The hydrogels of the invention can be obtained by a method, hereinafter "the method for obtaining the hydrogels of the invention" or "method of the invention". Thus, another aspect of the invention relates to a method for obtaining the hydrogels of the invention comprising the following steps:

   a) mixing Col I, agarose, DS, HA and EL, obtaining a solution (i),
   b) mixing Col I, agarose, DS, HA, obtaining a solution (ii), and
   c) putting the solution (i) and the solution (ii) in contact, obtaining a bilaminar hydrogel.

Preferably, the bilaminar hydrogel obtained in step c) is the bilaminar hydrogel of the invention.

In another preferred embodiment of the method of the invention, agarose is previously heated to a temperature of 60 to 80°C, preferably 70°C before mixing with the rest of the components in steps a) and b) of the method. More preferably, agarose is previously heated to a temperature of 60 to 80°C, preferably 70°C, and later tempered, preferably at a temperature of, at least, 37°C, more preferably tempered at a temperature of 37 to 40°C (including the end values), before mixing with the rest of the components in steps a) and b) of the method.

The term "tempered", as used herein, refers to a reduction or moderation of the temperature to which agarose is heated.

In another preferred embodiment of the method of the invention, step b) further comprises mixing EL with the rest of the components.

In another preferred embodiment of the method of the invention, the solutions (i) and (ii) are filtered through a filter or porous membrane before step c) of the method of the invention. More preferably, the filter comprises a pore size of 0.22 $\mu$m.

In another preferred embodiment of the method of the invention, alone or in combination with the other preferred embodiments:

- the concentration of Col I is from 1 to 3.5 mg/ml (including the end values), the concentration of DS is from 7 to 10 mg/ml (including the end values), the concentration of HA is from 0.5 to 1.5 mg/ml (including the end values), the concentration of Ag is from 10 to 30 mg/ml (including the end values), and the concentration of EL is from 0.5 to 1.5 mg/ml (including the end values) in the solution (i); and

- the concentration of Col I is from 1 to 3.5 mg/ml (including the end values), the concentration of DS is from 7 to 10 mg/ml (including the end values), the concentration of HA is from 0.5 to 1.5 mg/ml (including the end values), the concentration of Ag is from 10 to 30 mg/ml (including the end values), and the concentration of EL is from 0.5 to 1.5 mg/ml (including the end values) in the solution (ii).

In another more preferred embodiment of the method of the invention:

- the concentration of Col I is 2.2 mg/ml, the concentration of DS is 8.4 mg/ml, the concentration of HA is 1 mg/ml, the concentration of Ag is 15 mg/ml, and the concentration of EL is 1 mg/ml in the solution (i); and

- the concentration of Col I is 2.2 mg/ml, the concentration of DS is 8.4 mg/ml, the concentration of HA is 1 mg/ml, and the concentration of Ag is 15 mg/ml in the solution (ii).

An example of a suitable source of DS and HA for the purpose of the present invention includes the product Dermial ®, containing both components. Thus, in another preferred embodiment of the method of the invention, alone or in combination with the other preferred embodiments, Dermial ® is used as a source of DS and HA.

Furthermore, as mentioned previously, some hydrogels of the invention may comprise cells, preferably human cells. Thus, in another preferred embodiment of the method of the invention, step a) further comprises adding DFs, preferably human DFs (hDFs), and step b) further comprises adding MSCs, preferably human MSCs (hMSCs). In another preferred embodiment, alone or in combination with the other preferred embodiments, the concentration of MSCs is 1M/ml and the concentration of DFs is 1M/ml.

The terms "mesenchymal stem cells" and "dermal fibroblasts" have been previously explained in another aspect of the invention, and both they and their preferred embodiments are applicable to the present aspect of the invention.

In a preferred embodiment of the method for obtaining the invention, alone or in combination with the other preferred embodiments, in step (c) which comprises putting the solution (i) and the solution (ii) in contact, both solutions are added sequentially. More preferably, the solution (ii) is added after the solution (ii). Even more preferably, the sequential addition is carried out by a syringe.

In another embodiment of the method for obtaining the bilaminar hydrogel of the invention, alone or in combination with the other preferred embodiments, the bilaminar hydrogel is cultured at a temperature of 30 to 40°C (including the end values), preferably at 37°C. In another preferred embodiment of the method for obtaining the bilaminar hydrogel of the invention, the bilaminar hydrogel is cultured at a temperature of 30 to 40°C in the presence of 5% $CO_2$.

Furthermore, to obtain some hydrogels of the invention, the method of the invention may comprise additional steps. Thus, a preferred embodiment of the method for obtaining the hydrogels of the invention further comprises the following additional steps:

(d) mixing Col I with Kt and Sph, obtaining a solution (iii), and
(e) putting the solution (iii) in contact with the bilaminar hydrogel obtained in c), obtaining a trilaminar hydrogel.

Preferably, the trilaminar hydrogel obtained in step e) of the method of the invention is the trilaminar hydrogel of the invention.

A preferred embodiment of the method of the invention, alone or in combination with the other preferred embodiments, comprises an additional step of gelification. Said gelification step is carried out after the bilaminar hydrogel is obtained or after the trilaminar hydrogel is obtained.

The term "gelling", as used in this document, refers to a process in which a solution or mixture of components is transformed into a solid, semi-solid or semi-liquid state under conditions in which different forces or interactions of the components that form it are produced through, for example, hydrogen bonds, Van der Waals forces, covalent, ionic, supramolecular bonds and combinations thereof.

In a preferred embodiment of the method of the invention, the gelification comprises:

- a first substep of gelification, which comprises incubating the bilaminar hydrogel or the trilaminar hydrogel obtained in the method of the invention at a temperature below 37°C for 1 to 5 minutes (including the end values), and
- a second substep, after the first gelification substep, comprising incubating the bilaminar hydrogel or the trilaminar hydrogel at a temperature of 37°C, preferably for 15 to 120 minutes (including the end values).

In a preferred embodiment of the method of the invention, alone or in combination with the other preferred embodiments, the concentration in the solution (iii) of Col I is from 3.5 to 5.5 mg/ml (including the end values), the concentration of Kt is from 10 to 20 mg/ml (including the end values) and the concentration of Sph is from 2.5 to 7.5 mg/ml (including the end values).

In another more preferred embodiment of the method of the invention, alone or in combination with the other preferred embodiments, the concentration in the solution (iii) of Col I is 4.4 mg/ml, the concentration of Kt is 15.2 mg/ml and the concentration of Sph is 5 mg/ml.

As already mentioned in other aspects of the invention, some hydrogels of the invention may comprise EKs. Thus, another preferred embodiment of the method for obtaining the hydrogels of the invention, alone or in combination with the other preferred embodiments, further comprises adding EKs, preferably human EKs (hEKs). In a more preferred embodiment, the EKs are added to the trilaminar hydrogel obtained in step (e) or after the gelification step. In another even more preferred embodiment, the added EKs are allowed to culture for 0.5 to 3 weeks, more preferably 1 week. In another preferred embodiment, alone or in combination with the other preferred embodiments, the concentration of EKs is $1M/cm^2$.

On the other hand, the hydrogels of the invention can be dehydrated, preferably partially dehydrated, which gives them properties that include, but are not limited to, greater resistance, stiffness or mechanical properties. Thus, another preferred embodiment of the method of the invention, alone or in combination with the other preferred embodiments, further comprises a step (f) of dehydrating the hydrogel, after a bilaminar hydrogel is obtained, or after a trilaminar hydrogel is obtained.

Preferably, the dehydration step comprises applying pressure, more preferably, applying 50 to 150 g of pressure, including the end values. Even more preferably, dehydration comprises applying 50, 55, 60, 65, 70, 75, 80, 85, 90, 95, 100, 105, 110, 115, 120, 125, 130, 135, 140, 145 or 150 g of pressure.

In another preferred embodiment, dehydration comprises applying pressure for 1 to 5 minutes. Preferably, it comprises applying pressure for 1, 2, 3, 4, or 5 minutes. More preferably, it comprises applying pressure for 2 minutes. Even more preferably, dehydration comprises applying 100 g of pressure for 2 minutes.

As previously described in other aspects of the invention, some hydrogels of the invention are lyophilised. Thus, in some preferred embodiments, the method of the invention further comprises a final step of lyophilisation of the hydrogel. The lyophilisation step comprises lyophilising the hydrogel, preferably it comprises lyophilising the bilaminar hydrogel or the trilaminar hydrogel without cells. The term "lyophilisation" has already been explained in previous aspects of the invention, and both it and its preferred embodiments are applicable to the method of the invention.

More preferably, lyophilisation of the hydrogel comprises freezing and subsequent application of vacuum.

In an even more preferred embodiment, freezing of the lyophilisation step is carried out at a temperature from -20°C to -80°C (including the end values), more preferably from - 60°C to -80°C (including the end values), even more preferably at -70°C.

In an even more preferred embodiment, alone or in combination with the foregoing preferred embodiments, the step for applying vacuum is carried out for 1.5 hours to 16 hours (including the end values).

The hydrogel obtained after the final step of lyophilisation is the lyophilised bilaminar hydrogel of the invention, or the lyophilised trilaminar hydrogel of the invention.

In another preferred embodiment, alone or in combination with the other preferred embodiments, the method of the invention comprises an additional freezing step, preferably after the dehydration step and prior to lyophilisation. Said freezing step comprises incubating the sample at a temperature below -10°C. Preferably, the freezing step is carried out at a temperature of -40°C to -10°C, including the end values. More preferably, freezing is carried out at a temperature of -40, -35, -30, -25, -20, -15, or - 10°C. Even more preferably, freezing is carried out at a temperature of -20°C.

As already mentioned, the hydrogels of the invention can be obtained by the method for obtaining the hydrogels of the invention.

Thus, another aspect of the invention relates to the bilaminar hydrogel of the invention obtained by the method of the invention.

Another aspect of the invention relates to the lyophilised bilaminar hydrogel of the invention obtained by the method of the invention.

Another aspect of the invention relates to the trilaminar hydrogel of the invention obtained by the method of the invention.

Another aspect of the invention relates to the lyophilised trilaminar hydrogel of the invention obtained by the method of the invention.

Some terms used to define the method for obtaining the hydrogels of the invention have already been explained previously and both they and their preferred embodiments are applicable thereto.

Method of treatment of the invention

In another aspect, the invention relates to a method for treating skin lesions or wounds in a subject, comprising the administration of one of the hydrogels of the invention, hydrogels of the invention, hydrogels obtained by the obtaining method of the invention, bio-inks of the invention, pharmaceutical composition of the invention, dressing or implant of the invention to said subject.

Another aspect of the invention relates to a method for regenerating, repairing or replacing skin tissue in a subject, comprising the administration of one of the hydrogels of the invention, hydrogels of the invention, hydrogels obtained by the obtaining method of the invention, bio-ink of the invention, pharmaceutical composition of the invention, dressing or implant of the invention to said subject.

In a preferred embodiment, the subject is a mammal, more preferably a primate, even more preferably a human being.

References to *in vivo* treatment methods by therapy in this description are to be understood as references to the substances, pharmaceutical compositions and medicaments of the present invention for use in such methods.

The terms defined and explained for the rest of the aspects of the invention, as well as the preferred embodiments thereof, are also applicable to the present aspect of the invention.

## DESCRIPTION OF THE FIGURES

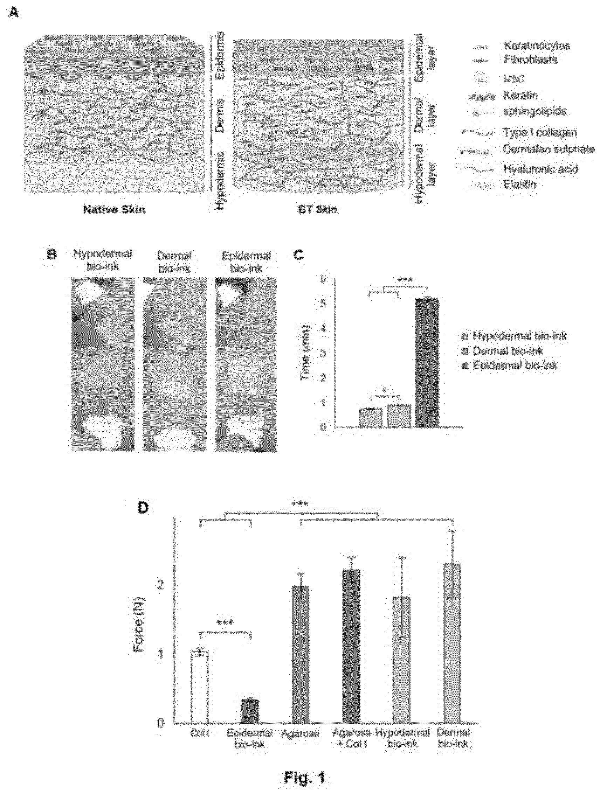

**Fig. 1.** (A) Design of the BT Skin hydrogel compared to native skin. Cells are represented as keratinocytes, fibroblasts and mesenchymal stem cells (MSCs), and biological components such as keratin, sphingolipids, type I collagen, dermatan sulphate, hyaluronic acid and elastin. (B) Tube inversion test images and (C) gel time results of each hypodermal, dermal and epidermal bio-ink. (D) Minimum force required to extrude the bio-inks through a 3 ml bioprinter syringe. Statistical significance: * P <0.05; *** P <0.005.

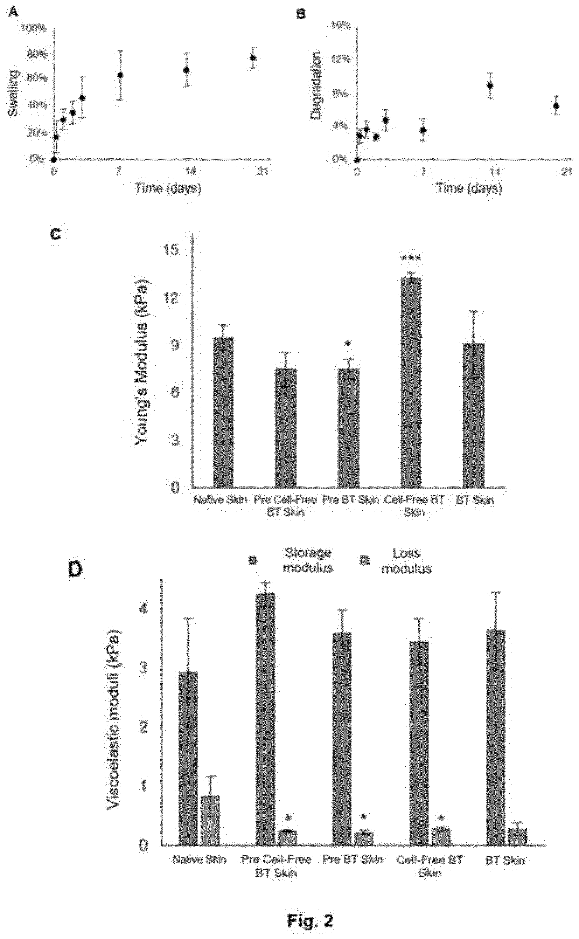

**Fig. 2.** Swelling (A), degradation (B) and rheological (C and D) assays. (A) Swelling behaviour and (B) percentage of degradation of the BT Skin hydrogel during 21 days. Young's modulus (C) and viscoelastic moduli (D) of BT Skin hydrogels, with or without cells, before and after the partial dehydration process, after 21 days in culture, compared to native skin. Statistical significance: * P <0.05, *** P <0.005.

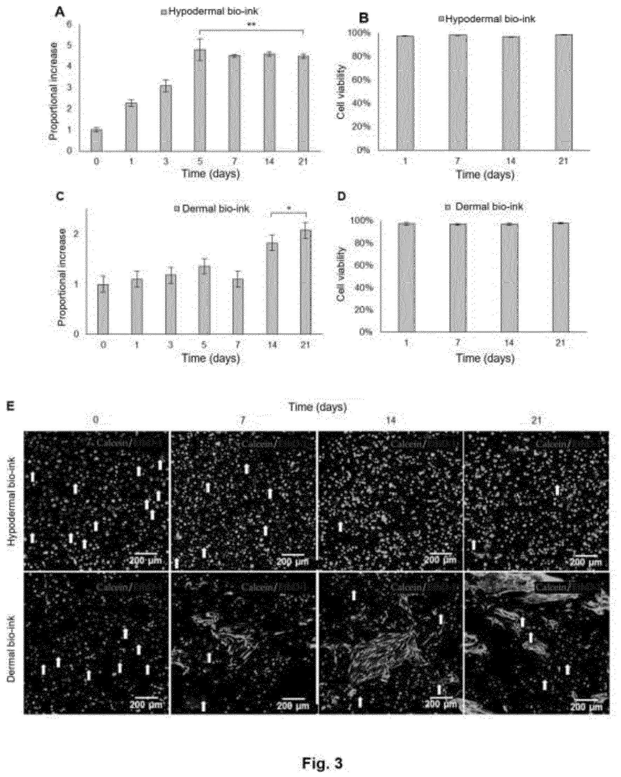

**Fig. 3.** Cell proliferation (A) and viability (B) of hypodermal bio-ink. Cell proliferation (C) and viability (D) of the dermal bio-ink. (E) Representative microscopy images of the hypodermal and dermal bio-inks on days 0, 7, 14, and 21. A live/dead assay was used, using calcein and ethidium homodimer; live cells were stained with calcein while dead cells were stained with ethidium homodimer. The white arrows point to dead cells, and the unmarked cells are live cells. Scale bars: 200 $\mu$m.

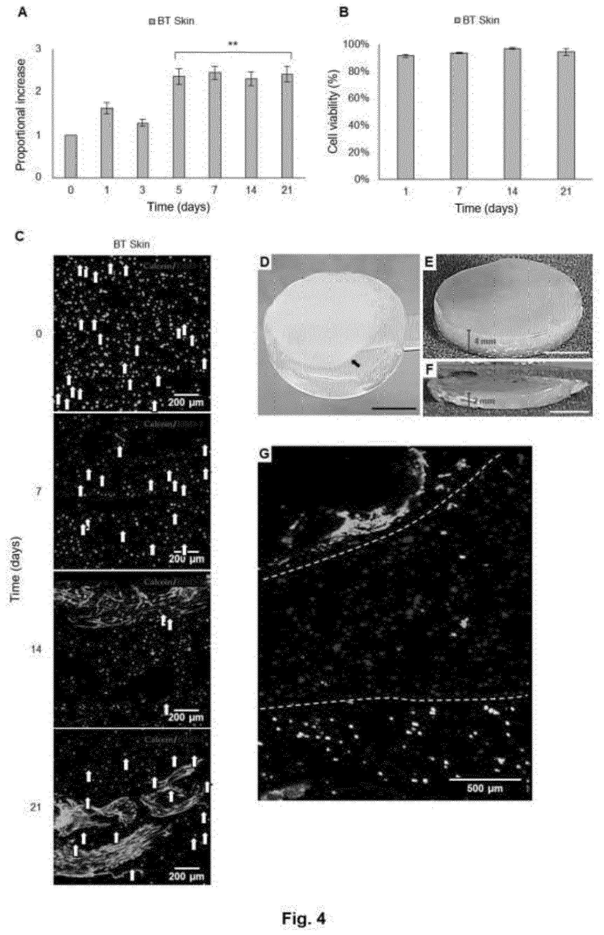

**Fig. 4.** Cell proliferation (A) and viability (B and C) of the BT Skin hydrogel (the white arrows point to dead cells, and the unmarked cells are live cells. Scale bars: 200 $\mu$m). Macroscopic images of the BT Skin hydrogel (D; black arrow: epidermal layer), before (E) and after (F) partial dehydration (scale bars = 5 mm). (G) Confocal fluorescence image of the BT Skin hydrogel after 21 days of culture (hEKs labelled at the top, hDFs labelled in Cell Tracker Red in the middle and hMSCs labelled in Cell Tracker Green at the bottom).

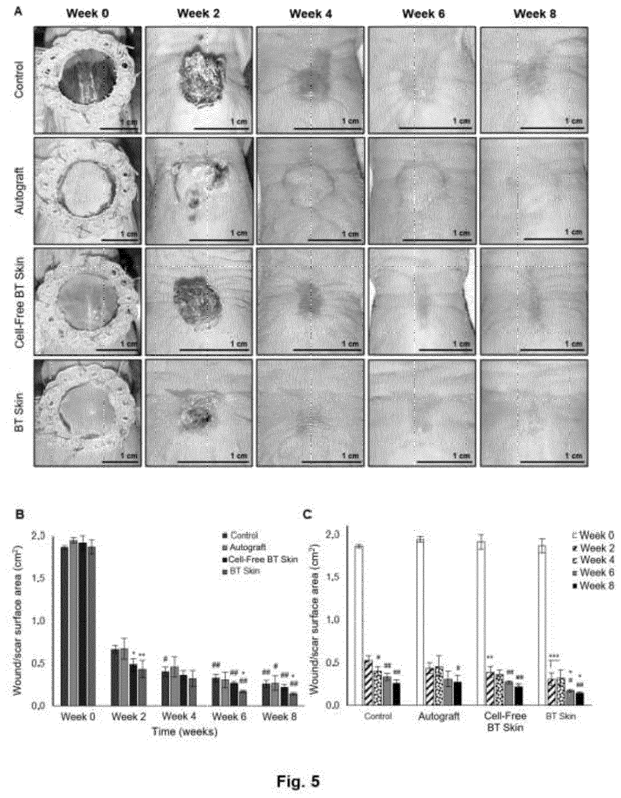

**Fig. 5.** Macroscopic images of the wound healing process over time (A). The type of treatment is indicated in each row, while the progression time (week 0, 2, 4, 6, 8) is represented in each column. Scale bars are represented on each image: 1 cm. Quantitative assessment of wound/scar surface area over time for all the groups (B) and in separate groups (C). Statistical significance compared to the control: * P <0.05; ** P <0.01; *** P <0.005. Statistical significance compared to day 14: #P <0.05; ## P <0.01.

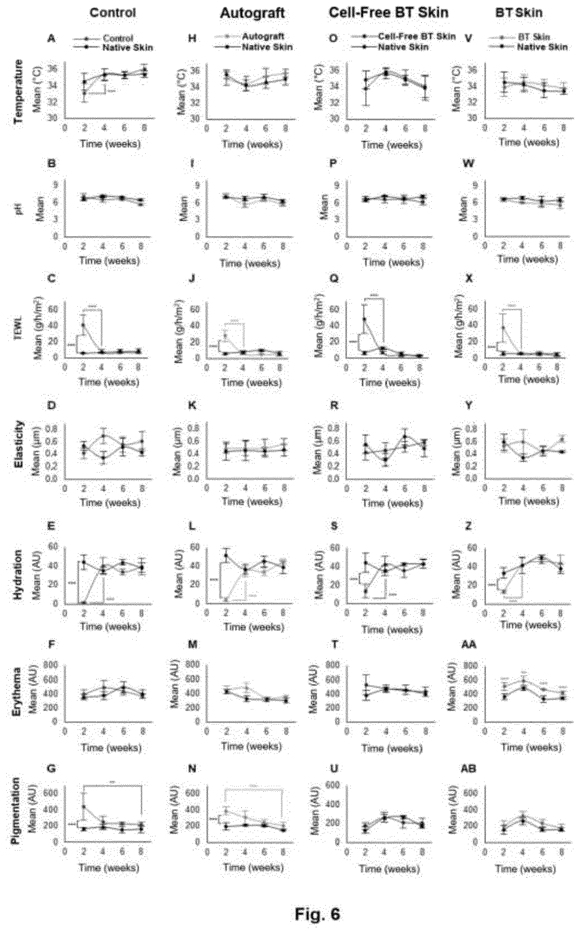

**Fig. 6.** Analysis of homeostasis parameters by week and group. The graphs show the results of each treatment group compared to the native skin group. (A, B, C, D, E, F and G) Control group; (H, I, J, K, L, M and N) Autograft group; (O, P, Q, R, S, T and U) Cell-Free BT Skin; (V, W, X, Y, Z, AA and AB) BT Skin. The results per week were calculated as the mean value of all the measurements of the mice at each moment of the study; Control, autograft, cell-free BT Skin and BT Skin groups (n weeks 2, 4, 6, 8 = 8, 8, 4, 4); Native skin group (n weeks 2, 4, 6, 8 = 32, 32, 16, 16). Statistical significance: * P <0.05; ** P <0.01; *** P <0.005.

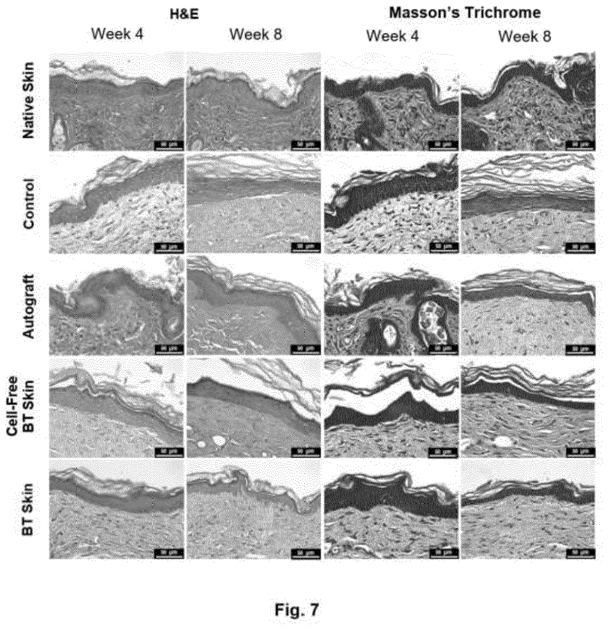

**Fig. 7.** Histological stains with hematoxylin and eosin (H&E) and Masson's Trichrome from biopsies of the wound/scar area of mice and native skin after 4 and 8 weeks. Scale bar: 50 $\mu$m.

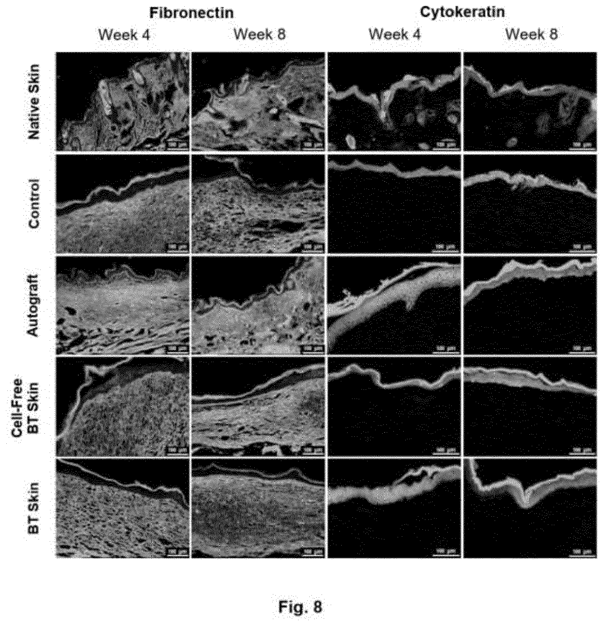

**Fig. 8.** Fibronectin and cytokeratin profiles of the wound/scar area of mice and native skin biopsies after 4 and 8 weeks. Fluorescent microscopy observations. Scale bar: 100 $\mu$m.

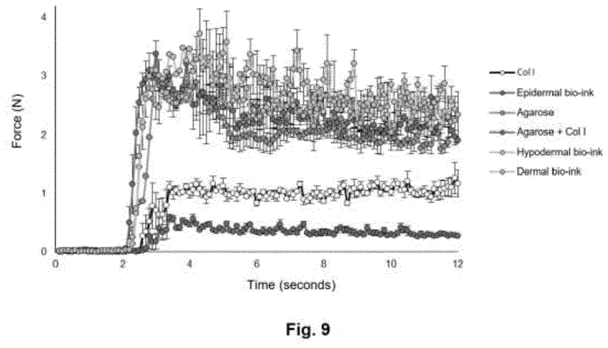

**Fig. 9.** Injectability of the base bio-inks (Col I and Agarose) and studied bio-inks (epidermal, hypodermal and dermal).

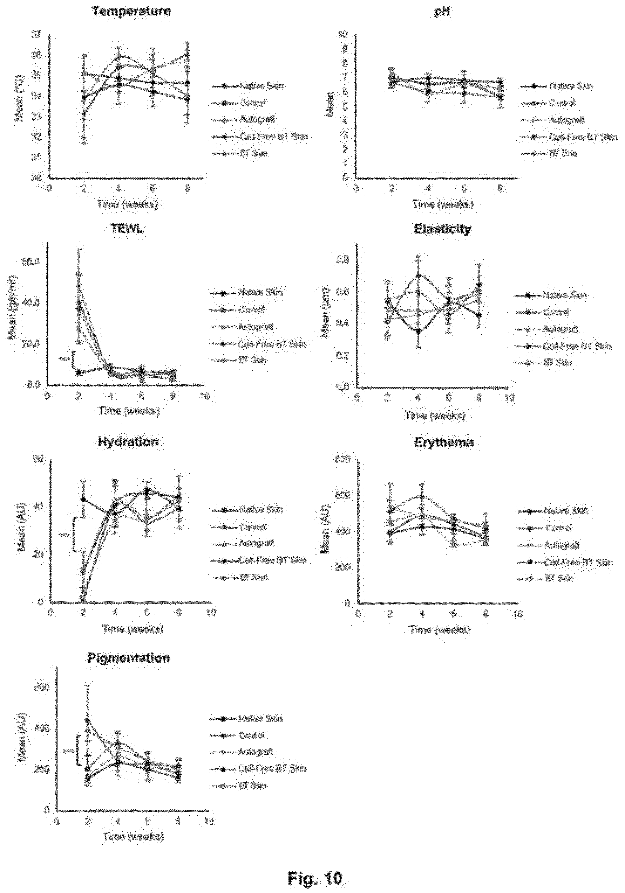

**Fig. 10.** Analysis of homeostasis by parameter. The graphs show the results of each treatment group compared to the native skin group. The results per week were calculated as the mean value of all the measurements of the mice at each moment of the study; Control, autograft, cell-free BT Skin and BT Skin groups (n weeks 2, 4, 6, 8 = 8, 8, 4, 4); Native skin group (n weeks 2, 4, 6, 8 = 32, 32, 16, 16). Statistical significance: *** P <0.005.

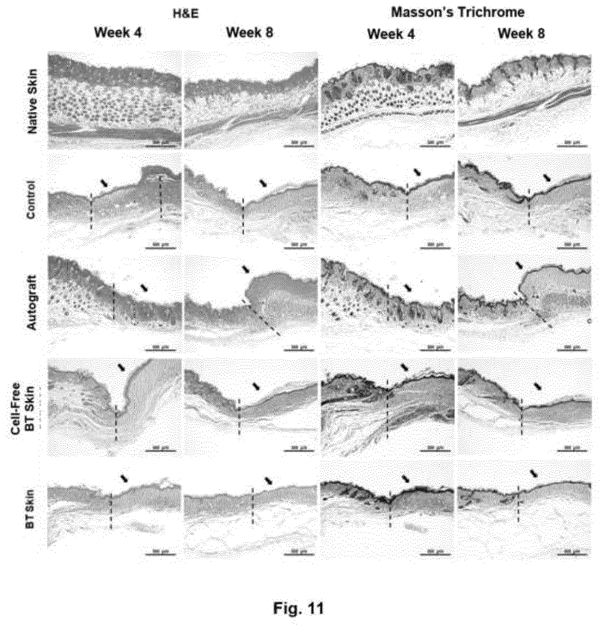

**Fig. 11.** Histological stains with hematoxylin and eosin (H&E) and Masson's Trichrome from biopsies of the wound/scar area of mice and native skin after 4 and 8 weeks. Scale bar: 500 $\mu$m.

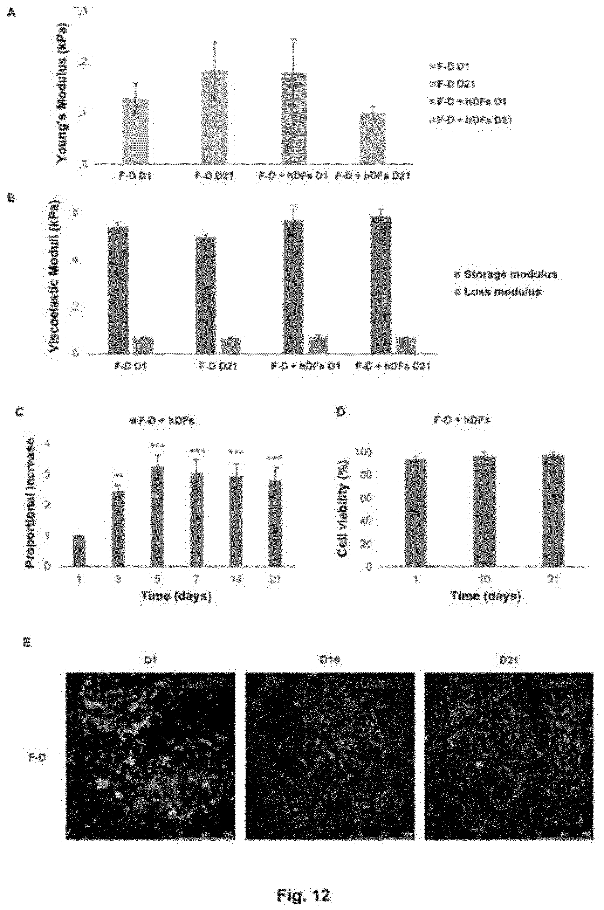

**Fig. 12.** Lyophilised (F-D). (A) Compression and (B) Viscoelasticity. Cell proliferation (C) and viability (D). (E) Confocal fluorescence images after 21 days. D: days.

**Fig. 13.** (A) Macroscopic images of the wound healing process over time. Type of treatment indicated in each row, time progression indicated in each column (weeks 0, 2, 4, and 8). The scale bars represented in each image: 1 cm. (B) Quantitative assessment of wound/scar area over time for all conditions, and (C) by condition. Statistical significance compared to the control: ***P < 0.005. Statistical significance compared to day 14: #P < 0.05; ##P < 0.01.

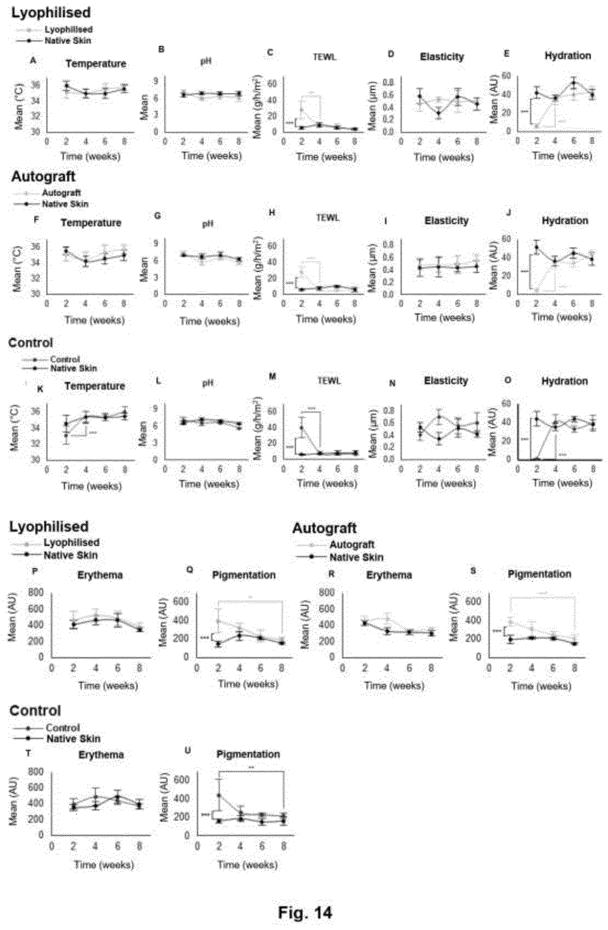

**Fig. 14.** Analysis of homeostasis parameters by week and group. The graphs show the results of each group compared to those of native skin. (A, B, C, D, E, P, Q) Lyophilised dressing group, (F, G, H, I, J, R, S) autograft group, and (K, L, M, N, O, T, U) control group. The results per week were calculated as the mean of all mouse measurements at each time point; Lyophilised, autograft, control (n per week 2, 4, 6, 8 = 24, 24, 12, 12); native skin (n per week 2, 4, 6, 8 = 8, 8, 4, 4). Statistical significance: *P < 0.05; **P < 0.01; ***P < 0.005.

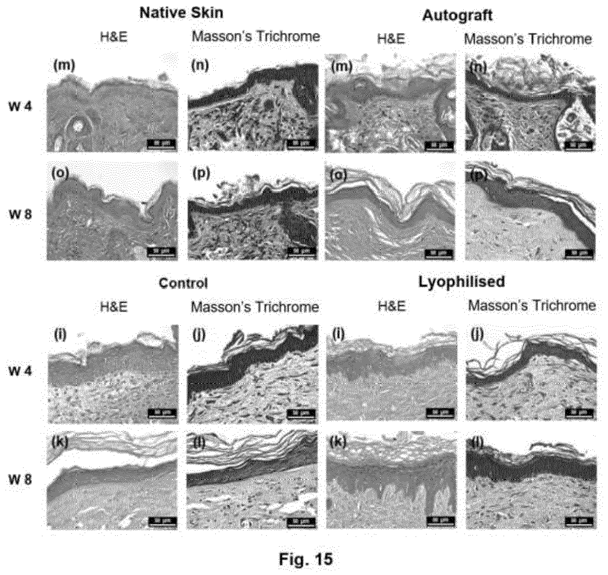

**Fig. 15.** Histological stains of hematoxylin & eosin (H&E) and Masson's Trichrome from mouse skin biopsies in the wound/scar area and native skin after 4 and 8 weeks. Scale: 50 $\mu$m.

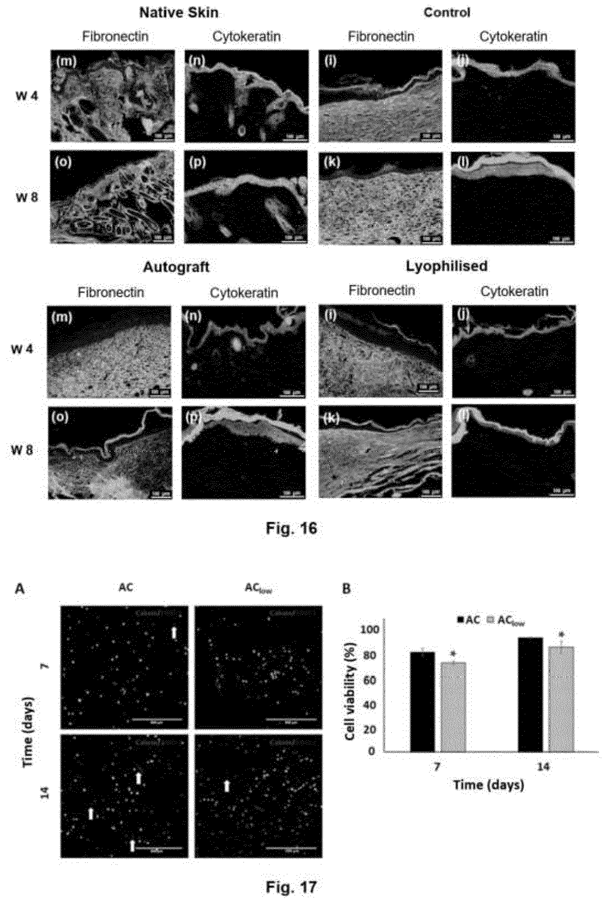

**Fig. 16.** Fibronectin and cytokeratin profiles from mouse skin biopsies in the wound/scar area and native skin after 4 and 8 weeks. Fluorescent microscopy observations. Scale: 100 $\mu$m.

**Fig. 17.** Cell viability of $AC_{low}$ and AC hydrogel formulations. (A) Confocal images of the fibroblast-laden hydrogels after 7 and 14 days. Calcein marks live cells and ethidium heterodimer marks dead cells. The white arrows point to dead cells, and the unmarked cells are live cells. Scale = 500 $\mu$m. (B) Cell viability (%) in the hydrogels after 7 and 14 days. A two-tailed Student's t-test analysis was performed to calculate the significance of the samples from the $AC_{low}$ and AC hydrogels at levels of significance of: * p < 0.05.

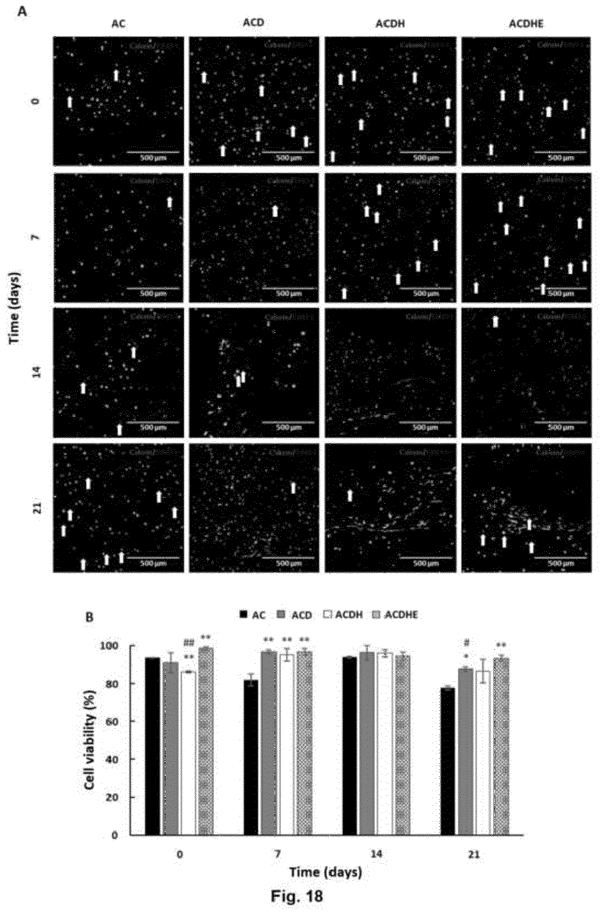

**Fig. 18.** Cell viability assay of AC, ACD, ACDH and ACDHE hydrogel samples. (A) Confocal microscopy images of hDF-laden hydrogels at 0, 7, 14, and 21 days. Calcein stains live cells, while EthD-1 stains dead cells. The white arrows point to dead cells, and the unmarked cells are live cells. Scale bar = 500 $\mu$m. (B) Cell viability (%) on the hydrogel scaffolds after 0, 1, 7, 14 and 21 days. Two-tailed Student's t-test analyses were performed for ACD, ACDH, ACDHE samples compared to AC samples at each time point at levels of significance of: * p < 0.05 and ** p < 0.01; and for ACDHE samples compared to ACD, ACDH samples at each time point at levels of significance of: # p < 0.05 and ## p < 0.01.

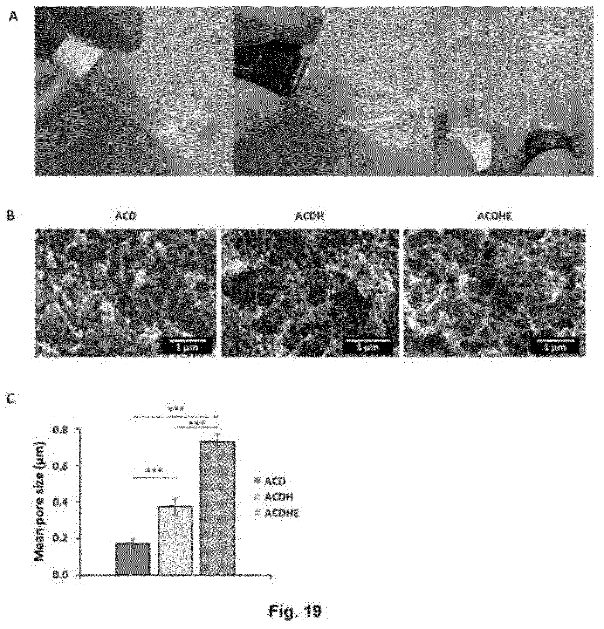

**Fig. 19.** Tube inversion test and ultrastructure of the hydrogels. (A) Representation of AC hydrogels (white cap) and ACDHE hydrogels (black cap) withstanding the tube inversion test. (B) ESEM images (scale bar = 1 $\mu$m) and (C) pore size characterisation of ACD, ACDH and ACDHE hydrogels. Two-tailed Student's t-test analyses were performed for the ACDH and ACDHE samples compared to the ACD samples with a level of significance of: *** $p < 0.05$.

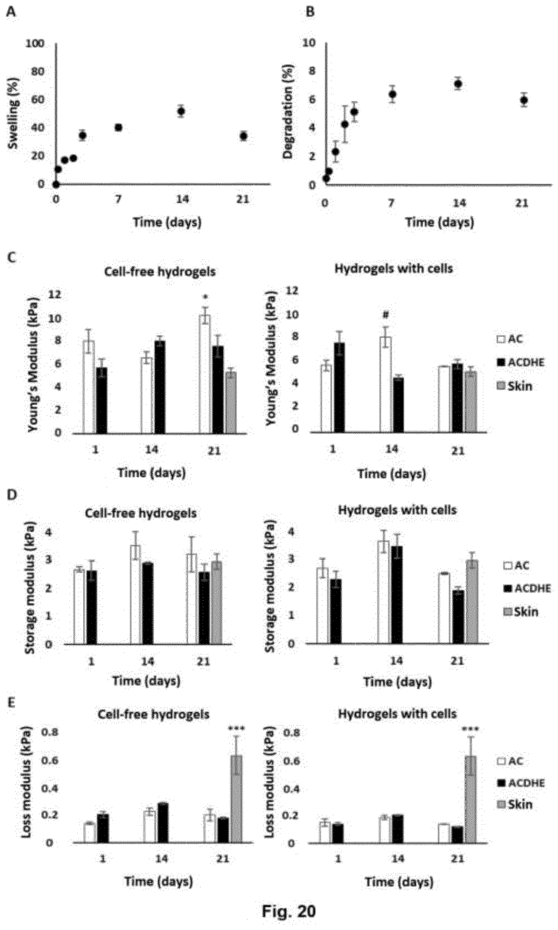

**Fig. 20.** Swelling, degradation and mechanical assays. (A) Swelling behaviour and (B) degradation rates of the ACDHE hydrogel over a 21-day time span. (C-E) Mechanical measurements: (C) Young's Moduli, (D) storage moduli, and (E) loss moduli of AC and ACDHE hydrogels cell-free and with cells at 1, 14 and 21 days under culture conditions, compared to native human skin. Two-tailed Student's t-test analyses were performed for human skin samples compared to AC and ACDHE samples on day 21 at levels of significance of: * $p < 0.05$ and *** $p < 0.005$; and for AC samples compared to ACDHE samples at each time point at a level of significance of: # $p < 0.05$.

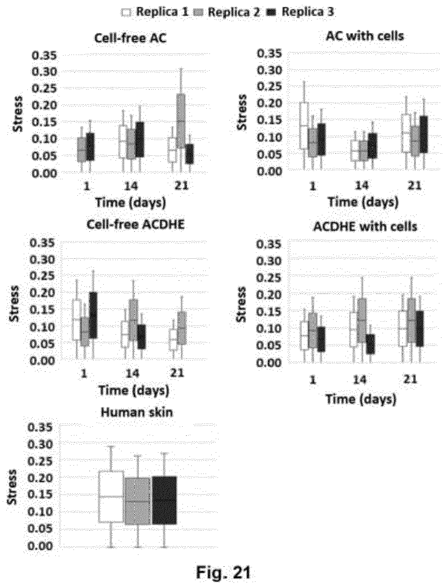

**Fig. 21.** Stress range analysed to obtain the Young's modulus of hydrogels with and without AC and ACDHE cells, and from human skin samples.

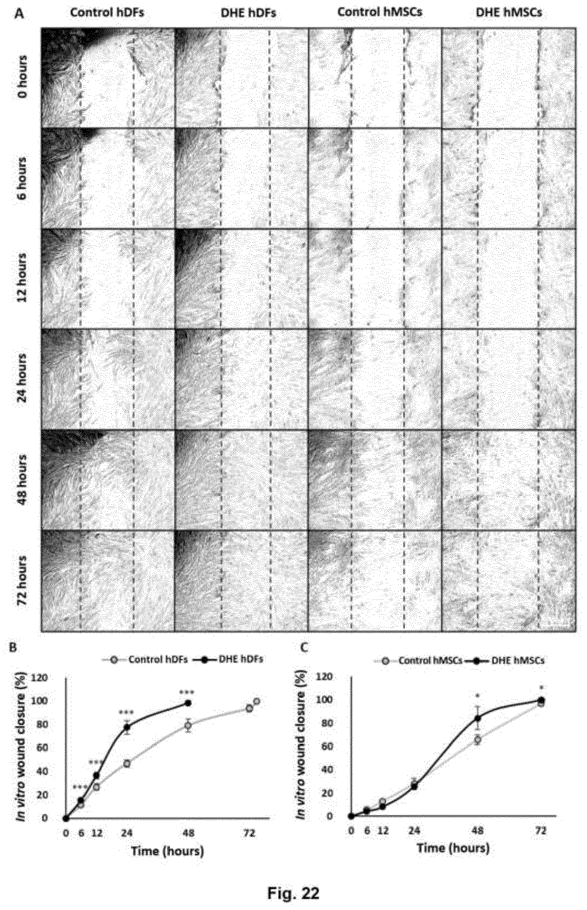

**Fig. 22.** *In vitro* wound healing assay of hDFs and hMSCs treated with DHE-supplemented media. A culture medium without supplements was used as a control for the untreated cells. The images (A) and the analysed results (B, C) showed wound closure at 0, 6, 12, 24, 48 and 72 hours. Statistical significance: * $p < 0.05$; *** $p < 0.005$. Abbreviations: DHE, dermatan sulphate, hyaluronic acid and elastin; MSC, mesenchymal stem cells.

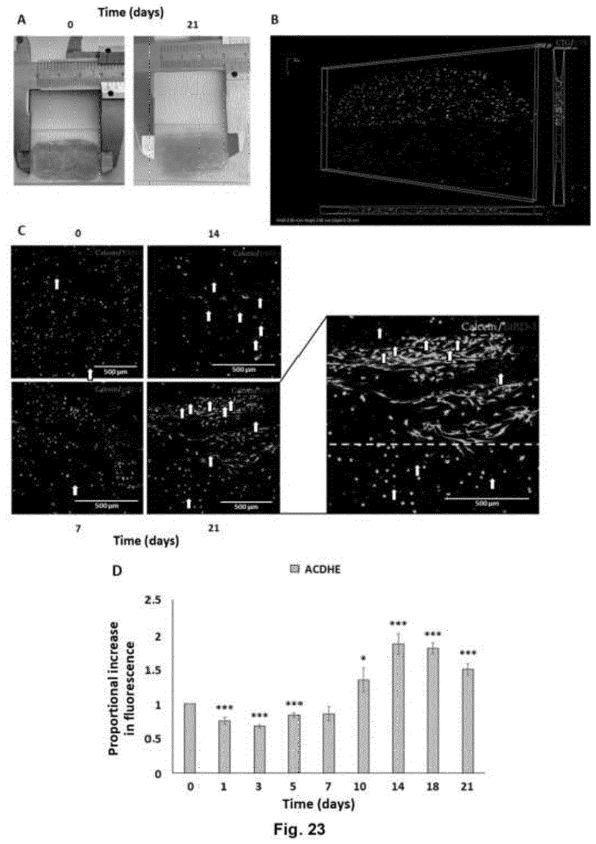

**Fig. 23.** ACDHE bilaminar hydrogel. (A) Macroscopic appearance of bilaminar ACDHE hydrogels. (B) Vertical cross-sectional image of the bilaminar hydrogel acquired by confocal microscopy (the white arrows point to dead cells, and the unmarked cells are live cells. Scale bars: 200 $\mu$m). Side and bottom views are also included on the right and bottom sides of the figure. hDFs and hMSCs are stained with CellTracker™ Green CMFDA (CTG) and Cell-Tracker™ Red CMTPX (CTR), respectively. Scale bar = 1000 $\mu$m. (C) Confocal microscopy images of cell viability of the ACDHE hydrogel on days 0, 7, 14, and 21. Additionally, the enlarged image of the bilaminar ACDHE hydrogel is shown on day 21, the white dotted line separates the fibroblast layer (top) from the hMSCs layer (bottom). Scale bar = 500 $\mu$m. (D) Results of proliferation of the ACDHE hydrogels up to 21 days.

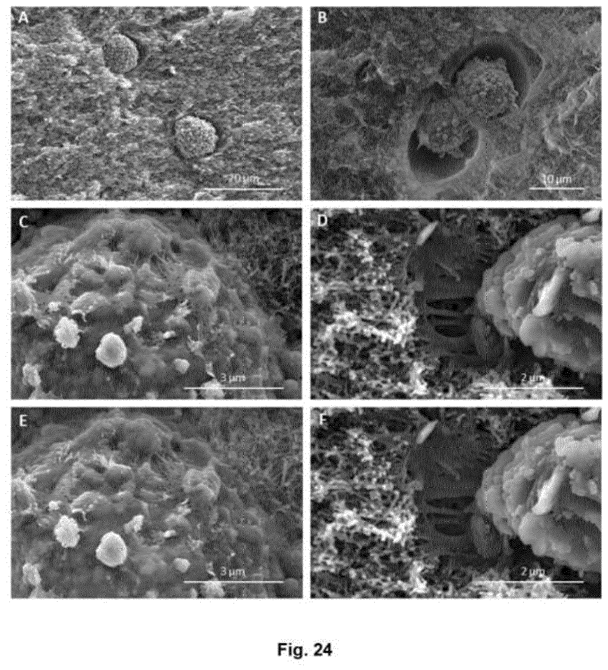

**Fig. 24.** ESEM of bilaminar ACDHE hydrogel. (A-F) ESEM images (scale bar = 1 $\mu$m) of ACDHE hydrogels. Scale bars: (A) 20 $\mu$m; (B) 10 $\mu$m; (C and E) 3 $\mu$m; (D and F) 2 $\mu$m. (E and F). ESEM images showing cells and structures similar to ECM produced by cells.

## EXAMPLES

Next, the invention will be illustrated by means of assays carried out by the inventors which demonstrate the effectiveness of the invention.

## 1. Materials and methods

### 1.1. Trilaminar model

#### 1.1.1. Cell cultures

Human mesenchymal stem cells (hMSC) were isolated from human adipose tissue and characterised using a method already described (López-Ruiz E, et al., Eur Cells Mater., 35:209-24 (2018); Gálvez-Martín P, et al., Eur J Pharm Biopharm., 86(3):459-68 (2014)). All human samples used in this study were obtained with informed consent and the approval of the Institutional Review Board (ethical permission number: 02/022010 Virgen de la Victoria Hospital, Malaga, Spain), and transported to the laboratory in Dulbecco's Modified Eagle Medium (DMEM; Sigma, St. Louis, MO, USA) with 1% penicillin and streptomycin (P/S; Invitrogen). hMSCs were cultured in high-glucose DMEM supplemented with 10% foetal bovine serum (FBS) and 1% P/S, at 37°C in a humidified atmosphere containing 5% $CO_2$. The medium was renewed every 3 days. At 80% confluence, the cells were subcultured and used between passages 4 and 6 for all experiments.

Human dermal fibroblasts (hDF) and human epidermal keratinocytes (hEK) were isolated from human skin samples obtained from donors using a previously published method (Sierra-Sánchez Á, et al., J Eur Acad Dermatology Venereol. (2020)). Once the skin samples were removed, they were transported to the laboratory in sterile containers containing high-glucose DMEM without phenol red supplemented with antibiotics. All human skin samples were obtained in accordance with the ethical standards of the committee responsible for human experimentation (Provincial Ethical Committees of Granada) and with the Declaration of Helsinki of 1975, revised in 1983. Once isolated, the hDFs and hEKs were cultured (Carriel V. et al., Cells Tissues Organs, 196196:1-121(2012)).

## 1.1.2. Preparation of bio-inks and the BT Skin substitute

Hydrogel-based bio-inks were prepared to create a trilaminar skin substitute (also called BT Skin).

First, an agarose (Ag) solution (UltraPure™ Low Melting Point Agarose Thermo Scientific™) was prepared in phosphate-buffered saline (PBS), sterilised in an autoclave at 120°C for 2 hours and stored at 4°C until use. The Ag was preheated and kept at 37°C in a water bath to avoid gelling and to stabilise its temperature at 37°C. The Col I solution (3.3 mg/ml rat tail collagen I, Corning®) was neutralised with 0.8M NaHCOs. Next, three solutions were prepared using Kt (Kerapro S, Proalan S.A., Barcelona, Spain), Sph, DS, HA and EL (Bioibérica S.A.U, Barcelona, Spain) diluted in PBS: i) Kt + Sph (KS); ii) DS + HA (DH); iii) DS + HA + EL (DHE). To include DS and HA, Dermial ®, a product that has both active ingredients, was used. Each solution was mixed with Col I to the final concentration as shown in Table 1. All component solutions were filtered through a 0.22 μm membrane (Merck Millipore) before use.

Table 1. Concentrations of agarose, collagen I, dermatan sulphate (DS), hyaluronic acid (HA), elastin (EL), keratin (Kt) and sphingolipids (Sph) in the different bio-inks.

| Composition (mg/ml) | Agarose | Collagen I | DS | HA | EL | Kt | Sph |
|---|---|---|---|---|---|---|---|
| Epidermal bio-ink | - | 4.4 | - | - | - | 15.2 | 5.0 |
| Dermal bio-ink | 15 | 2.2 | 8.4 | 1.0 | 1.0 | - | - |
| Hypodermal bio-ink | 15 | 2.2 | 8.4 | 1.0 | - | - | - |

To biomanufacture the BT Skin substitute, cell-laden dermal and hypodermal bio-inks were prepared by mixing Col I + DHE with hDFs and Col I + DH with hMSCs, respectively, and preheated Ag was added to each mixture, obtaining a final concentration of $1 \cdot 10^6$ cells/ml. Once both the hypodermal and dermal layers were obtained, the epidermal bio-ink (Col I + KS) was placed on top and left to gel in an incubator at 37°C for 15 minutes. Once the epidermal layer gelled, $2 \cdot 10^6$ hEKs were sown on the BT Skin hydrogel and cultured for 1 week. After this time, the samples were partially dehydrated by applying 100 g of pressure for 2 minutes, giving the hydrogels greater resistance and enhancing their stiffness and mechanical properties.

## 1.1.3. Physico-chemical characterisation of trilaminar hydrogels

Tube inversion test

The tube inversion test was used to define the gel time of the bio-inks. The bio-inks were poured into glass vials, inverting the vials upside down every 1 minute to check for the formation of stable gels. The gel time was considered to be the time when the samples formed a stable gel that remained on the bottom of the vials when inverted.

pH

The pH of the hydrogel samples was determined using a Hach Sension + calibrated digital pH meter (Hach Lange S.L., Spain) at room temperature (RT).

Iniectabilitv test

The force required to extrude the different biomaterial mixtures was measured using an MCR302 torsional rheometer (Anton Paar, Austria). The equipment used for the assay was arranged as shown in Fig. 1D, where a 3 ml syringe was kept surrounded by hot water at a temperature of 37°C to prevent rapid gelification and to reproduce the environment of a heated syringe with a 3D bioprinter. The samples were placed in the syringe and the force required to extrude the bio-inks was controlled.

Swelling test

The swelling rates of the lyophilised samples were analysed. Briefly, the samples were previously weighed and immersed in PBS. The swelling rate of the hydrogel samples was calculated at different times as follows:

$$Swelling\ rate\ (\%) = \left(\frac{(W_t - W_0)}{W_0}\right) x\ 100$$

$W_0$ represents the initial weight of the samples on day zero and Wt represents the wet weight of the samples at the corresponding time.

Degradation test

The degradation rate was analysed by quantifying the weight loss of the samples over time. The hydrogels were incubated with gentle stirring at 37°C, recovered at different times and centrifuged at 5000 rpm for 2 minutes. The supernatant was removed and the samples were weighed. The degradation rate (%) was calculated as a measure of weight loss as follows:

$$Degradation\ rate\ (\%) = \left(\frac{(W_t - W_i)}{W_i}\right) x\ 100$$

$W_i$ represents the initial weight of the samples and $W_t$ represents the wet weight of the hydrogels at the corresponding time.

Mechanical analysis

Rheological tests were carried out using an MCR302 torsional rheometer. The hydrogel samples were moulded using a 20 mm-diameter and 5 mm-height mould. A three-step assay was designed to obtain data on the compression and shear characteristics of the hydrogels. In the first step, the samples were placed on the base of the rheometer, approaching the head of the rheometer at 10 $\mu$m/s up to a normal force of 0.5 N. Secondly, the samples were subjected to oscillatory shearing with a strain amplitude of 0.00001% at a frequency of 1 Hz and a normal force of 0.5 N to analyse the viscoelastic shear moduli, and finally the top plate was withdrawn at a constant speed of 10 $\mu$m/s.

**1.1.4. Cell viability assay**

The LIVE/DEAD™ Viability/Cytotoxicity Kit (Invitrogen, Massachusetts, USA) was used to analyse cell viability. The hydrogels were stained using a solution of Calcein-AM (2 $\mu$M; green) and ethidium homodimer (4 $\mu$M; red) diluted in PBS at 37°C for 30 minutes. The samples were observed using confocal microscopy (Nikon Eclipse Ti-E A1) at different times and analysed using NIS-Elements software. The live and dead cells were quantified using imaged software (Fiji), determining the percentage of cell viability as follows:

$$Cell\ viability\ (\%) = \frac{Live\ cells}{(Live\ cells\ + Dead\ cells\ )} x\ 100$$

**1.1.5. Cell proliferation assays**

The AlamarBlue HS® assay (Invitrogen) was used to analyse cell proliferation of the samples after 1, 3, 5, 7, 14 and 21 days of culture. Fluorescence was measured after incubation, which gave rise to a signal that may be related to the metabolic activity of the cells. The samples were incubated with the AlamarBlue HS® solution at 37°C for 1 hour. The fluorescence of the reduced solution was determined at excitation/emission wavelengths of 530/590 nm.

**1.1.6. *In vivo* assay**

1.1.6.1. Wound healing animal model, surgical procedures and experimental groups

A total of 32 male and female ATHYM-Foxn1nu/nu, immunocompromised, athymic, hairless and albino 4-week-old mice were used for the *in vivo* assay. All animal handling procedures followed national and European Union legislation (RD 53/2013 and EU Directive 2010/63) on the protection of animals used for scientific purposes and in accordance with the Ethical Principles and Guidelines for the Use of Animals approved by the Provincial Ethical Committees of Granada.

Surgery was performed to remove a 2 cm$^2$ area of skin from the upper dorsal part, in a position longitudinal to the mouse's spinal column, using surgical scissors. A 3D-printed splint using 1,4-butanediol thermoplastic polyurethane elastomer (b-TPUe), porous, donut-shaped, and designed with a hinged cover was centred over the wound and secured with seven interrupted sutures. Then, the mice were transplanted with BT Skin, cell-free BT Skin, lower back skin graft (Autograft), or left untreated as control conditions (Control) (n=8 per group). Lastly, the cover of the splint was closed with eight interrupted sutures. Samples and splints were grafted for all the groups, and an antibiotic ointment was applied (Mupirocin 20 mg/g; ISDIN). Likewise, an analgesic (Bupredine 0.3 mg/ml 10 ml; Fatro Ibérica, Desvern, Barcelona, Spain) and an antibiotic (Ganadexil Enrofloxacin 5% 100 ml; Industrial Veterinaria, S.A. Invesa) were injected subcutaneously as post-operative treatment.

### 1.1.6.2. Healing monitoring

Follow-up was carried out for 8 weeks, gathering clinical information, such as scar/wound surface area, and several homeostasis parameters. Furthermore, after 8 weeks, the scars were assessed using an adaptation of the Patient and Observer Scar Assessment Scale (POSAS). Every 2 weeks, mice were anaesthetised by isoflurane inhalation to avoid unnecessary stress and cutaneous homeostasis parameters were measured by the Microcaya probe system (Microcaya SL, Bilbao, Spain), allowing the monitoring of different cutaneous parameters: the Thermometer® probe allowed skin temperature to be measured in °C; the Skin pH-meter® probe measured skin pH; the Tewameter® probe determined transepidermal water loss (TEWL), such as the evaporation of water in g/h/m$^2$; the Cutometer® probe analysed the elasticity of skin ($\mu$m) with suction (450 mbar of negative pressure - 2 s); the Corneometer® probe determined skin hydration through the capacitance of a dielectric medium; and the Mexameter® probe, based on the absorption/reflection of light in three wavelengths, was able to measure erythema and skin pigmentation, obtaining indirect information on vascularisation (haemoglobin levels) and pigmentation (melanin), respectively. The values of healthy skin were obtained by measuring a healthy area of skin (native skin) from each mouse in the study.

### 1.1.6.3. Histological analysis and immunostaining

Four and eight weeks later, the mice were sacrificed by cervical dislocation once anaesthetised by isoflurane inhalation. Graft biopsies and native skin samples were collected, fixed in 4% paraformaldehyde, dehydrated, embedded in paraffin or in an optimal cutting temperature (OCT) compound (Tissue-Tek®, Sakura Finetek) and cut into 5 $\mu$m or 8 $\mu$m sections using a microtome and cryostat, respectively.

The paraffin sections were deparaffinised, rehydrated and stained with hematoxylin and eosin (H&E) and Masson's Trichrome to reveal the histological structure. For immunofluorescence analysis, the cryosections were incubated with primary antibodies against fibronectin (Santa Cruz Biotechnology, 1:100) and cytokeratin (Invitrogen, 1:100). Next, the sections were incubated with Alexa-488 conjugated anti-rabbit secondary antibody (ThermoFisher, 1:500) and counterstained with Hoechst. The images were obtained using a Leica DM 5500B microscope and analysed using imaged software.

### 1.2. Lyophilised trilaminar model

To manufacture the lyophilised trilaminar model, dermal and hypodermal bio-inks were prepared by mixing Col I + DHE and Col I + DH, and prewarmed Ag was added to each mixture. Once both the hypodermal and dermal layers were obtained, the epidermal bio-ink (Col I + KS) was placed on top and left to gel in an incubator at 37°C for 15 minutes. Once the epidermal layer gelled, the samples were partially dehydrated by applying 100 g of pressure for 2 minutes, giving the hydrogels greater resistance and enhancing their stiffness and mechanical properties. Lastly, the samples were frozen at -20°C and they were introduced into the lyophiliser which cold trap was previously cooled to -70°C. The lyophilisation chamber was closed, keeping the samples at room temperature and a vacuum was applied using a pressurised air pump. The samples were kept in the lyophilisation process until it could be macroscopically observed that they had been properly dehydrated (between 2-16 hours).

### 1.2.1. Biological and mechanical characterisation of lyophilised dressings

For this lyophilised trilaminar model, mechanical analyses, cell viability assays, and cell proliferation assays were carried out under the same experimental conditions as for the non-lyophilised trilaminar model.

**1.2.2. *In vivo assays***

Furthermore, as in the non-lyophilised trilaminar model, a series of *in vivo* assays were carried out.

Wound healing animal model, surgical procedures and experimental groups

A total of 24 male and female ATHYM-Foxn1nu/nu, immunocompromised, athymic, hairless and albino 4-week-old mice were used for the *in vivo* assay.

Surgery was performed to remove a 2 cm$^2$ area of skin from the upper dorsal part, in a position longitudinal to the mouse's spinal column, using surgical scissors. A 3D-printed splint using 1,4-butanediol thermoplastic polyurethane elastomer (b-TPUe), porous, donut-shaped, and designed with a hinged cover was centred over the wound and secured with seven interrupted sutures. Then, the mice were transplanted with the lyophilised dressing, lower back skin graft (Autograft), or left untreated as control conditions (Control) (n=8 per group). Lastly, the cover of the splint was closed with eight interrupted sutures. Samples and splints were grafted for all the groups, and an antibiotic ointment was applied (Mupirocin 20 mg/g; ISDIN). Likewise, an analgesic (Bupredine 0.3 mg/ml 10 ml; Fatro Ibérica, Desvern, Barcelona, Spain) and an antibiotic (Ganadexil Enrofloxacin 5% 100 ml; Industrial Veterinaria, S.A. Invesa) were injected subcutaneously as post-operative treatment.

Healing monitoring

Follow-up was carried out for 8 weeks, gathering clinical information, such as scar/wound surface area, and several homeostasis parameters. Every 2 weeks, mice were anaesthetised by isoflurane inhalation to avoid unnecessary stress and cutaneous homeostasis parameters were measured by the Microcaya probe system (Microcaya SL, Bilbao, Spain), allowing the monitoring of different cutaneous parameters: the Thermometer® probe allowed skin temperature to be measured in °C; the Skin pH-meter® probe measured skin pH; the Tewameter® probe determined transepidermal water loss (TEWL), such as the evaporation of water in g/h/m2; the Cutometer® probe analysed the elasticity of skin ($\mu$m) with suction (450 mbar of negative pressure -2s); the Corneometer® probe determined skin hydration through the capacitance of a dielectric medium; and the Mexameter® probe, based on the absorption/reflection of light in three wavelengths, was able to measure erythema and skin pigmentation, obtaining indirect information on vascularisation (haemoglobin levels) and pigmentation (melanin), respectively. The values of healthy skin were obtained by measuring a healthy area of skin (native skin) from each mouse in the study.

Histological analysis and immunostaining

Four and eight weeks later, the mice were sacrificed by cervical dislocation once anaesthetised by isoflurane inhalation. Graft biopsies and native skin samples were collected, fixed in 4% paraformaldehyde (PFA), dehydrated, embedded in paraffin or in an optimal cutting temperature (OCT) compound (Tissue-Tek®, Sakura Finetek) and cut into 5 $\mu$m or 8 $\mu$m sections using a microtome and cryostat, respectively.

The paraffin sections were deparaffinised, rehydrated and stained with hematoxylin and eosin (H&E) and Masson's Trichrome to reveal the histological structure. For immunofluorescence analysis, the cryosections were incubated with primary antibodies against fibronectin (Santa Cruz Biotechnology, 1:100) and cytokeratin (Invitrogen, 1:100). Next, the sections were incubated with Alexa-488 conjugated anti-rabbit secondary antibody (ThermoFisher, 1:500) and counterstained with Hoechst. The images were obtained using a Leica DM 5500B microscope and analysed using imaged software.

**1.3. Bilaminar model**

**1.3.1. Cell cultures**

hDFs were obtained from the American Type Culture Collection (ATCC® PCS-201-012) and cultured in Dulbecco's Modified Eagle Medium (DMEM; Sigma) containing 10% foetal bovine serum (FBS; Sigma), 100 U/ml penicillin and 100 mg/ml streptomycin (Invitrogen) at 37°C in a 5% $CO_2$ humidified atmosphere. The medium was changed every 3 days. When 80% confluence was reached, the cells were released with Tryple Express (Gibco) and subcultured. hDFs were used between passages 4 and 6 for all experiments.

hMSCs were obtained from human adipose tissue and characterised using a previously described method (López-Ruiz E, et al., Eur Cells Mater., 35:209-24 (2018); Gálvez-Martin P, et al., Eur J Pharm Biopharm., 86(3):459-68 (2014)). hMSCs were cultured in high-glucose DMEM (Sigma) supplemented with 10% FBS, 100 U/ml penicillin and 100 mg/ml streptomycin (Invitrogen) at 37°C in a humidified atmosphere containing 5% $CO_2$. The medium was changed every 3 days. At 80% confluence, the cells were subcultured. hMSCs were used between passages 4 and 6 for all experiments.

### 1.3.2. Hydrogel formulation

To prepare the hydrogel, two different concentrations of Ag were dissolved, a previously reported Ag-Col I (AC) hydrogel formulation (Ag 1.5% w/v) and an AC hydrogel with a reduced agarose concentration (1.2% w/v) (AClow). The Ag solutions were prepared by dissolving UltraPure Low Melting Point Agarose (Thermo Scientific) in a phosphate buffer (PBS). This solution was autoclaved at 120°C for 2 hours and stored at 4°C. Before its use, Ag was preheated to 70°C and kept in a 37°C water bath to warm and prevent gelling. The Col I solution (4.42 mg/ml rat tail collagen I, Corning) was kept on ice before use and neutralised with 0.8 M NaHCOs. The pH of the Col I solution after neutralisation was 7.4. Next, three stock solutions were prepared using DS, HA and EL (Bioibérica SAU) diluted in PBS (DS; DS + HA; DS + HA + EL) and mixed with Col I at the final concentrations shown in Table 2. The Col I solutions were filter sterilised through a 0.22 $\mu$m pore size filter (Merck Millipore) before use. Similarly, DS, HA and EL lyophilised powder was sterilised using UV radiation for at least 30 minutes. Finally, cell-laden hydrogels were prepared by mixing the aforementioned solutions with hDFs or hMSCs and prewarmed Ag was added to the mixture, obtaining 1.1 ml of hydrogel with 1.106 cells/ml.

Table 2. Concentration of the components in the bilaminar hydrogel. AC: agarose + collagen; ACD: agarose + collagen + dermatan sulphate; ACDH: agarose + collagen + dermatan sulphate + hyaluronic acid; ACDHE: agarose + collagen + dermatan sulphate + hyaluronic acid + elastin.

| (mg/ml) | Agarose | Collagen | Dermatan sulphate | Hyaluronic acid | Elastin |
|---|---|---|---|---|---|
| AC | 0.015 | 2.2 | - | - | - |
| ACD | 0.015 | 2.2 | 8.4 | - | - |
| ACDH | 0.015 | 2.2 | 8.4 | 1.0 | - |
| ACDHE | 0.015 | 2.2 | 8.4 | 1.0 | 1.0 |

### 1.3.3. Physical characterisation of the ACDHE hydrogel

Inversion test

To analyse the gelling of the hydrogel, the tube inversion test was performed and the AC and ACDHE formulations were assayed. The hydrogel mixture was poured into a glass vial for the preparation of the hydrogels. To check the formation of a stable gel, the vial was inverted upside down every 1 minute and the gel time was noted.

pH analysis

The pH values of the hydrogels generated at room temperature were determined using a Hach Sension+ calibrated digital pH meter (Hach Lange S.L.) at 25.0 $\pm$ 0.5°C. Measurements were made by direct contact of the pH meter electrode in hydrogels.

Environmental Scanning Electron Microscopy (ESEM)

ESEM was used to observe the morphology of the surface of the hydrogels and the internal cell distribution. The hydrogel samples were fixed with 2.5% w/v glutaraldehyde (Merck) for 1 hour at 4°C, then they were washed and maintained in 0.1 M sodium cacodylate buffer (EMS, Electron Microscopy Science). Before their analysis, the samples were processed using the critical point drying technique. Four samples were fixed with 1% w/v osmium tetroxide, dehydrated in a series of ethanol solutions of increasing concentration (50, 70, 90 and 100%) for 15 minutes each, and dried at the critical point in a Leica EM CPD300 dryer. Lastly, the samples were covered with carbon using the EMITECH K975X carbon evaporator. Images were acquired with ESEM QEMSCAN 650F and hydrogel pore sizes were analysed with imaged software (Fiji).

Swelling assay

Pre-weighed lyophilised hydrogels were immersed in PBS. The weight of the prepared hydrogels was calculated at different times. The swelling ratio was calculated with the following equation:

$$Swelling\ rate\ (\%) = \left(\frac{(W_t - W_0)}{W_0}\right) x\ 100$$

Where W0 represents the dry weight of the sample on day zero and Wt represents the wet weight of the samples at a specific time.

Degradation test

The degradation behaviour of the hydrogels was analysed by weighing known amounts of hydrogel samples. Next, the hydrogel samples were incubated with gentle stirring in a hybridisation oven at 37°C. The samples were recovered at different times from the hybridisation oven and centrifuged at 5000 rpm for 2 minutes. After removing the supernatant, the hydrogel samples were finally weighed. The degradation rate (%) as a measure of weight loss was calculated with the following equation:

$$Degradation\ rate\ (\%) = \left(\frac{(W_t - W_0)}{W_0}\right) x\ 100$$

Where $W_0$ represents the initial weight of the sample and $W_t$ represents the wet weight of the samples at a specific time.

Mechanical assays

Mechanical analyses were performed in an MCR302 torsional rheometer (Anton Paar) at 25°C. Cell-free and hDF-laden hydrogels were cast into cylindrical shapes in moulds (20 x 5 mm). A three-step test was designed to obtain information on the compression and shear characteristics of the samples. First, the samples were placed on the base of the rheometer. Then, the rheometer head was approached at a constant speed (10 $\mu$m/s) up to a normal force of 0.5 N. Next, the sample was oscillatory sheared according to a strain amplitude of 0.00001% at a frequency of 1 Hz and a normal force of 0.5 N to determine the viscoelastic shear moduli and, lastly, the top plate was separated at a constant speed (10 $\mu$m/s).

**1.3.4. Wound closure assay**

hDFs and hMSCs were seeded in 12-well multiwell plates and cultured at 37°C for 72 hours. The culture medium was removed, and the cells were rinsed with PBS and incubated with a culture medium supplemented with 8.4 mg/ml DS, 1.0 mg/ml HA and 1.0 mg/ml elastin for 24 hours. A culture medium without supplements was used as an untreated control condition. After 24 hours, the medium was removed, the cells were washed with PBS and a trace was made on the cell monolayer by manual scratching with a 200 $\mu$l pipette tip. The supplemented and control media were replaced, and images of the wounds were taken at 0, 6, 12, 24, 48 and 72 hours. The samples were kept at 37°C in a 5% $CO_2$ atmosphere. The images were taken with a Leica DMi8 microscope (Leica Microsystems) with Leica Application Suite (LAS) X software and analysed with imaged software (Fiji).

**1.3.5. Cell viability assay**

Cell viability was determined using the LIVE/DEAD™ Viability/Cytotoxicity Kit (Invitrogen). The samples were incubated in PBS containing calcein-AM (2 $\mu$M) and ethidium homodimer (4 $\mu$M) at 37°C for 30 minutes to stain live cells (green) and dead cells (red). Images of hydrogels were obtained by confocal microscopy at different times and analysed with NIS-Elements software (Nikon Eclipse Ti-E A1). The live and dead cells were counted using imaged software (Fiji), and cell viability was determined as follows:

$$Cell\ viability\ (\%) = \frac{live\ cells}{(live\ cells + dead\ cells)} x\ 100$$

### 1.3.6. Cell proliferation assay

Cell proliferation was analysed using the AlamarBlue® assay (Invitrogen) on days 1, 3, 5, 7, 14 and 21. This reagent is a solution of resazurin, a non-fluorescent cell-permeable blue compound that is modified by the reducing environment of viable cells in resorufin, a fluorescent red compound. Fluorescence can be measured after incubation and, therefore, the generated signal related to the metabolic activity of the samples. The hydrogels were incubated with AlamarBlue® solution at 37°C for 3 hours. The fluorescence of reduced AlamarBlue® was determined at excitation/emission wavelengths of 530/590 nm.

### 1.3.7. Bilaminar hydrogel design

Separate hMSC- or hDF-laden hydrogels were prepared in conical tubes and loaded into sterile 3 ml syringes. Then, the hydrogel layers were sequentially stacked, placing the hMSC layer at the bottom and the hDF layer at the top. The bilayer hydrogels were left to gel and then placed in a 4-well rectangular multiwell plate with DMEM containing 10% FBS and 1% penicillin/streptomycin and cultured at 37°C in 5% $CO_2$ atmosphere.

### 1.3.8. Morphological characterisation of bilaminar hydrogels

Maintenance of the hydrogel shape was monitored during a culture period of 21 days. To observe the size of the hydrogels over time, three hydrogel samples were prepared and seeded with hDF and hMSC, immersed in culture medium and kept in an incubator. At pre-established time intervals (0, 7, 14 and 21 days), the hydrogels were recovered from the solution, excess medium was removed with filter paper, and the length and width of the samples were measured.

### 1.4. Statistical analysis

The results of this work are represented as mean $\pm$ standard deviation (SD). The differences between two sets of data were tested using the two-tailed Student's t-test for unpaired samples. The differences were considered statistically significant at $P < 0.05$ (* / #), $P < 0.01$ (** / ##) and $P < 0.005$ (*** / ###).

### 2. Results

### 2.1. Trilaminar model

### 2.1.1. Physico-chemical properties of the trilaminar hydrogel

Tube inversion test and pH

The gel time was analysed by applying the tube inversion test for the hypodermal, dermal and epidermal bio-inks. Fig. 1B shows representative images of the bio-inks in their liquid and gel forms. The average gel times of the bio-inks were $0.76 \pm 0.03$, $0.91 \pm 0.03$ and $5.22 \pm 0.09$ minutes (Fig. 1C), respectively. The pH value of the complete BT Skin hydrogel was $7.59 \pm 0.12$.

Bio-ink injectability

To characterise the injectability of bio-inks, the force required to extrude the solutions from a 3 ml syringe was measured. The results varied between 1.0 and 2.3 N (Fig. 1D and Figure 9), where the epidermal bio-ink ($0.34 \pm 0.03$ N) showed the lowest value, even less than the force required to extrude Col I ($1.04 \pm 0.04$ N). Regarding the hypodermal bio-ink ($1.82 \pm 0.57$ N) and dermal bio-ink ($2.30 \pm 0.49$ N), although no differences were shown between them or compared to their base mixture, Ag ($1.99 \pm 0.17$ N) and Ag + Col I ($2.22 \pm 0.19$ N), they showed a higher range of N ($1.25 - 2.79$ N) than Col I and the epidermal bio-ink ($0.31 - 1.08$ N).

Swelling and degradation behaviour of the BT Skin hydrogel

Lyophilised BT Skin hydrogels were immersed in PBS (pH 7.4) to observe their swelling behaviour. The swelling kinetics of BT Skin over an elapsed time of 21 days is shown in Fig. 2A. The mean swelling of the BT Skin hydrogel was $70 \pm 7\%$. BT Skin hydrogel (lyophilised) was shown to reach a plateau step after 7 days. To analyse the resistance of BT Skin hydrogel over time, a degradation assay was carried out measuring the change in weight up to 21 days (Fig. 2B). The results showed that the maximum degradation rate was reached after 14 days with $8.8 \pm 1.5\%$ mass loss.

Mechanical properties of BT Skin hydrogel

BT Skin hydrogels with and without cells before (Pre BT Skin and Pre Cell-free BT Skin) and after (BT Skin and Cell-free BT Skin) were generated and maintained at 37°C in a 5% $CO_2$ atmosphere. The Young's moduli of the hydrogels maintained up to 21 days compared to native human skin biopsies are represented in Fig. 2C. Although variations were found in Pre BT Skin (7.49 $\pm$ 0.62 kPa) and Cell-free BT Skin (13.24 $\pm$ 0.30 kPa) compared to native skin (9.46 $\pm$ 0.79 kPa), BT Skin (9.06 $\pm$ 0.21 kPa) did not show significant differences compared to the reference tissue.

The viscoelastic moduli of cell-laden and cell-free BT Skin hydrogels were also measured before and after dehydration and are shown in Fig. 2D. Although the four tested conditions were found to share the same loss modulus range of native skin (2.92 $\pm$ 0.91 kPa), only the loss modulus of BT Skin hydrogels (0.28 $\pm$ 0.10 kPa) did not show significant differences with respect to the loss modulus of natural skin (0.83 $\pm$ 0.33 kPa).

### 2.1.2. Biological characterisation of bio-inks and BT Skin hydrogel

A biological characterisation of the hypodermal and dermal bio-inks was carried out with cell proliferation and viability assays. Since the handling of the epidermal bio-ink was complicated, since it was fragile once gelled, this bio-ink was incorporated into the BT Skin hydrogel and the biological characterisation of the complete hydrogel was carried out. The cell proliferation rates of the hydrogels of the hypodermal and dermal bio-inks on days 0, 1, 3, 5, 7, 10, 14, 18 and 21 are shown in Fig. 3A and C, respectively. The hypodermal hydrogels showed an increase in cell proliferation on day 5, maintaining a plateau step until the end of the experiment. Likewise, the dermal hydrogels increased their cell proliferation from day 14, maintaining their level until the end of the experiment. Both conditions were able to maintain the level of cell viability above 97% for the duration of the assay (Fig. 3B, D and E), as demonstrated by the images acquired at 0, 7, 14 and 21 days.

The BT Skin hydrogel was prepared in three layers with the hypodermal layer at the bottom, a middle dermal layer, and the epidermal layer on the top (Fig. 1A), and it was partially dehydrated. Fig. 4D shows the macroscopic appearance of the BT Skin (black arrow indicating the epidermal layer), and Figs. 4E and F show the height difference of the hydrogel before and after the partial dehydration process, where the height of the hydrogel is reduced by 2 mm.

The skin cell proliferation rate and viability up to 21 days are shown in Fig. 4A and B, respectively. Similarly, for hypodermal and dermal bio-ink hydrogels, the BT Skin showed an increase in the cell proliferation rate from day 5, remaining at a plateau step until the end of the experiment. The cells were able to adhere and grow in contact with the surrounding cells in some areas of the hydrogels, as can be seen in Fig. 4C. Furthermore, BT Skin showed cell viability rates between 90.9 and 98%. To observe the distribution of the three cell types within the hydrogel, hMSCs, hDFs and hEKs were stained with CellTrackerTM Green CMFDA, CellTrackerTM Red CMTPX and CellTrackerTM Green CMFDA, respectively. It could be seen that the three layers of the hydrogel were well-differentiated (Fig. 4G).

### 2.1.3. *In vivo assays*

### 2.1.3.1. Clinical analysis

Suitable wound stabilisation was observed for all the mouse groups at 4 weeks, without complications (Fig. 5A). Wound resolution was faster in the case of the groups treated with Autograft, Cell-free BT Skin and BT Skin than in the Control group. Similarly, wound repair was faster and more effective in the case of the BT Skin group, while the Control, Autograft and Cell-free BT Skin groups experienced slower improvement. In the case of BT Skin, total skin repair was observed after 8 weeks.

The evaluation of the results by visual clinical observation (Fig. 5A) was correlated with the quantitative analysis of the wound/scar surface area (Fig. 5B and C), where significant differences were found between the Cell-free BT Skin and BT Skin groups compared to the control group at week 2, indicating a positive effect of these conditions on the wound healing process, but at the end of the experiment, only the BT Skin group showed significant differences with the Control group.

### 2.1.3.2. Study of homeostasis parameters

Temperature, pH, TEWL, elasticity and moisture were monitored (Fig. 6 and Fig. 10), comparing all the groups with the native skin of mice.

The results of temperature (Fig. 6A, H, O and V) and pH (Fig. 6B, I, P and W) showed a homogeneous evolution in all the groups in general of the study, without differences compared to native skin. The temperature values of all the groups during the study ranged from 31.8 to 36.6°C, overlapping the temperature range of native skin (34.0 - 36.0°C). Similarly, the pH ranges of all the groups (5.2 - 7.8) and native skin (6.4 - 7.3) also generally overlapped throughout the duration of the experiment.

TEWL (Fig. 6C, J, Q and X) showed a significant decrease after 2 weeks in all the groups (Fig. 10), reaching native skin levels. Regarding elasticity (Fig. 6D, K, R and Y), although the Control and BT Skin groups showed oscillatory behaviour throughout the experiment, there were no significant differences between all the groups and Native Skin in general at 8 weeks. Lastly, similar to the results of TEWL, the monitoring of moisture (Fig. 6E, L, S and Z) showed a recovery after 2 weeks, restoring native skin levels at 4 weeks in all the groups.

### 2.1.3.3. Erythema and pigmentation

The evaluation of erythema (Fig. 6F, M, T and AA) from the BT Skin groups of control, autograft and without cells did not show significant differences with respect to the native skin; however, BT Skin showed significantly higher levels during the 8 weeks of the experiment.

Regarding pigmentation (Fig. 6G, N, U and AB), while the Control and Autograft groups failed to achieve native skin melanin levels up to 4 weeks, after 2 weeks, the Cell-free BT Skin and BT Skin groups had already restored those levels.

### 2.1.3.4. Histological analysis and immunostaining

H&E and Masson's Trichrome stains of wound biopsies (Figure 7 and Fig. 11) showed correct regeneration of the epidermis and dermis after 4 and 8 weeks in all the groups; however, the Autograft, Cell-free BT Skin and BT Skin groups exhibited a more complex dermal matrix structure closer to native skin than the Control group, which showed a less dense dermal matrix structure after 4 weeks of surgical procedure.

As can be seen in Fig. 8, the immunostaining analysis showed the expression of fibronectin, a typical protein found in the abundantly expressed dermal extracellular matrix (ECM). Likewise, the expression of cytokeratin, a specific epidermal differentiation marker, was observed in all the groups.

### 2.2. Lyophilised trilaminar model

### 2.2.1. Mechanical properties

The samples with and without cells were generated and maintained at 37°C in a 5% $CO_2$ atmosphere. The Young's moduli of the samples kept up to 21 days are represented in Fig. 12A. Although variations were found between the 2 conditions in the period of 21 days, no significant differences were observed.

The viscoelastic moduli of the cell-laden and cell-free samples were also measured and are shown in Fig. 12B. It was observed that the two tested conditions shared a similar range of viscoelastic moduli, without showing significant differences therebetween during the 21 days.

### 2.2.2. Biological properties

A biological characterisation of the samples was carried out with cell proliferation and viability assays. The cell proliferation rates of the lyophilised dressings on days 1, 3, 5, 7, 14, and 21 are shown in Fig. 12C. The lyophilised dressings showed increased cell proliferation from day 3, maintaining a plateau step until the end of the experiment from day 5. The lyophilised dressings were able to maintain the level of cell viability above 93% for the duration of the assay (Fig. 12D and E), as demonstrated by the images acquired at 1, 10, and 21 days.

The BT Skin hydrogel was prepared in three layers with the hypodermal layer at the bottom, a middle dermal layer, and the epidermal layer on the top, and it was partially dehydrated. Fig. 4D shows the macroscopic appearance of the BT Skin (black arrow indicating the epidermal layer), and Figs. 4E and F show the height difference of the hydrogel before and after the partial dehydration process, where the height of the hydrogel is reduced by 2 mm.

### 2.2.3. *In vivo assays*

Evaluation of the healing process in mouse skin

Suitable wound stabilisation was observed for all the mouse groups at 4 weeks, without complications (Fig. 13A). Wound resolution was faster in the group treated with lyophilised dressing than in the Control group. Similarly, wound repair was faster and more effective in the case of this group, while the Control, Autograft and Cell-free BT Skin groups experienced slower improvement. The evaluation of the results by visual observation (Fig. 13A) was correlated with the quantitative analysis of the wound/scar surface area (Fig. 13B and C), where significant differences were found between the skin groups with lyophilised dressing compared to the control group at week 2, indicating a positive effect of this condition on the wound healing process.

Study of homeostasis parameters

Temperature, pH, TEWL, elasticity and moisture (Figure 3) were monitored, comparing all the groups with the native skin of mice. The results of temperature (Figures 14A, F, and K) and pH (Figures 14, G, and L) showed a homogeneous evolution in all the groups in general of the study, without differences compared to native skin. The temperature values of all the groups during the study ranged from 31.8 to 36.6°C, overlapping the temperature range of native skin (34.0 - 36.0°C). Similarly, the pH ranges of all the groups (5.2 - 7.8) and native skin (6.4 - 7.3) also generally overlapped throughout the duration of the experiment.

TEWL (Figures 14C, H, and M) showed a significant decrease after 2 weeks in all the groups, reaching native skin levels. Regarding elasticity (Figures 14D, I, and N), although an oscillatory behaviour was observed in the Control group throughout the experiment, there were no significant differences between all the groups and Native Skin in general at 8 weeks. Lastly, similar to the results of TEWL, the monitoring of moisture (Figures 14E, J, and O) showed a recovery after 2 weeks, restoring native skin levels at 4 weeks in all the groups.

Erythema and pigmentation

The evaluation of erythema (Figures 14P, R, and T) of the 3 groups did not show significant differences with respect to the native skin. Regarding pigmentation (Fig. 14Q, S, and U), the 3 groups reached native skin melanin levels after 4 weeks.

Histological analysis and immunostaining

H&E and Masson's Trichrome stains of wound biopsies (Fig. 15) showed correct regeneration of the epidermis and dermis after 4 and 8 weeks in all the groups; however, the Autograft and lyophilised groups exhibited a more complex dermal matrix structure closer to native skin than the Control group, which showed a less dense dermal matrix structure after 4 weeks of surgical procedure.

As can be seen in Fig. 16, the immunostaining analysis showed the expression of fibronectin, a typical protein found in the abundantly expressed dermal extracellular matrix. Likewise, the expression of cytokeratin, a specific epidermal differentiation marker, was observed in all the groups.

**2.3. Bilaminar model**

**2.3.1. Hydrogel formulation**

To determine the most suitable concentration of agarose, the cell viability of the AClow and AC hydrogels was investigated with the Live/Dead assay. Confocal images were used to calculate the percentage of cell viability. Although both concentrations showed good viability rates, cell viability in AClow hydrogels was significantly lower compared to AC hydrogels on days 7 and 14 (Fig. 17).

Furthermore, a loss of structural integrity was observed in AClow hydrogels after 7 days in culture. Thus, the referenced concentration of Ag (Köpf *et al.*, 8(2):025011 (2016)) was maintained in subsequent experiments.

To create a hydrogel that mimics the composition of the extracellular matrix (ECM) of the skin, AC-based hydrogels were supplemented with skin components. In other words, DS, a glycosaminoglycan found in native human skin; HA, an ECM polysaccharide from natural skin; and EL, a protein related to the ECM elasticity of the skin, were added to the AC formulation, generating three sequential formulations: ACD, ACDH and ACDHE.

The hDF-laden hydrogel solutions were pipetted onto the surface of a Petri dish, left to gel, gently transferred to a multiwell culture plate, and cultured for 21 days. All the conditions were mainly populated by live cells, showing a uniform distribution within the hydrogels (Fig. 18A).

The results of all the formulations did not show negative effects on the viability of the hDFs up to 21 days. The control and hydrogel formulations enriched with skin-related materials were able to maintain levels of viability greater than 78 and 86%, respectively (Fig. 18B), for at least 21 days. These results are consistent with the feasibility specifications for pharmaceutical products prior to their administration to patients, based on the primary quality criteria established by the Food and Drug Administration (FDA) and the European Medicines Agency (EMA) ($\geq$ 70% and $\geq$ 80%, respectively). ACDHE hydrogels showed higher levels of viability after 21 days compared to AC, ACD and ACDH hydrogels. The cell viability rates of the ACDHE condition were maintained above 93% throughout the experiment. Thus, the ACDHE hydrogel formulation with all ECM components was used for subsequent experiments.

### 2.3.2. Physico-chemical properties of the ACDHE hydrogel

Inversion test and pH

The time taken for the solution to turn into a gel was recorded using the tube inversion test. The mean gel times of the AC and ACDHE hydrogels were 3.4 ± 0.2 and 4.1 ± 0.2 minutes, respectively. Fig. 19A shows evidence of AC hydrogels (white cap vial) and ACDHE hydrogels (black cap vial) in their liquid and gel form. The pH value of the ACDHE hydrogels was 7.36 ± 0.05.

Hydrogel ultrastructure

To physically characterise the hydrogels, ESEM analysis was performed for the ACD, ACDH and ACDHE formulations. The three hydrogel formulations showed a uniform and homogeneous lattice organisation, with an interconnected porous network (Fig. 19B). The pore size increased proportionally with the complexity of the hydrogel formulation, the ACDHE hydrogel showing the largest pore size (Fig. 19C).

Swelling and degradation behaviours of the ACDHE hydrogel

Lyophilised ACDHE hydrogels were immersed in PBS (pH 7.4) to study the swelling behaviour of this formulation. The results of the swelling kinetics of the hydrogel are shown in Fig. 20A. The average swelling rate of the ACDHE hydrogel was found to be 42 ± 8%. The swelling kinetics of the ACDHE hydrogel indicated that a stationary phase was reached around 3 days after starting the assay, with a maximum peak on day 14. To study the stability of the ACDHE formulation, hydrogel degradation was determined by measuring the variation of weight over time up to 21 days (Fig. 20B). The maximum degradation rate was found after 14 days with almost 7.14 ± 0.22%.

Hydrogel mechanical properties

The mechanical properties of AC and ACDHE hydrogels were determined by mechanical assays. As stated in the materials and methods section, hydrogels without and with cells (hDFs) were placed in cylindrical moulds (20 mm in diameter and 5 mm in height) and kept at 37°C in a 5% $CO_2$ atmosphere. Fig. 20C shows the Young's moduli obtained from the compression assays of AC and ACDHE hydrogels up to 21 days in culture and they are compared with human skin.

Although slight variations were found between the Young's moduli of cell-free AC hydrogels and ACDHE hydrogels over time, the cell-free AC hydrogel after 21 days in culture (10.14 ± 0.72 kPa) showed significant differences compared to the range of elasticity observed in native human skin samples (5.22 ± 0.39 kPa), while no significant differences were found on day 21 of culture in cell-free ACDHE hydrogels (7.50 ± 0.94 kPa) compared to native human skin. On the other hand, in addition to the fact that the Young's moduli of the cell-laden hydrogels also showed variations over time, after 21 days of culture, neither the AC hydrogels (5.68 ± 0.00 kPa) nor the ACDHE hydrogels (5.86 ± 0.38 kPa) showed significant differences compared to native human skin (5.22 ± 0.39 kPa). This may be due to the fact that human skin tissue and hydrogels share a similar range of stiffness located in the kPa order of magnitude. The stress range analysed for the Young's moduli obtained in hydrogels and skin samples is shown in Fig. 21.

The viscoelastic moduli of the AC and ACDHE hydrogels, with cells and cell-free, are shown in Figs. 20D and Fig. 20E. Although the storage moduli of the AC and ACDHE hydrogels, either cell-free (3.19 ± 0.61 and 2.56 ± 0.29 kPa, respectively) or with cells (2.49 ± 0.03 and 1.89 ± 0.12 kPa, respectively) showed a slight variation over time, no significant differences were found after 21 days compared to native human skin samples (2.94 ± 0.27 kPa) (Fig. 20D).

On the other hand, as can be seen in Fig. 20E when analysing the loss moduli, significant differences were found on day 21 for the AC and ACDHE hydrogels, either cell-free (0.19 ± 0.04 and 0.17 ± 0.00 kPa, respectively) or hydrogels with cells (0.14 ± 0.00 and 0.12 ± 0.00 kPa, respectively) compared to native human skin samples (0.63 ± 0.13 kPa).

### 2.3.3. Wound closure assay

To study the wound healing effect of supplemented soluble biological compounds in the hydrogel formulation, a scratch wound assay was performed. Next, the dermatan/hyaluronic acid/elastin (DHE) solution was added to the culture media and studied in hDF and hMSC (Fig. 22A). Curiously, the hDFs cultured with DHE-supplemented media showed an important wound-healing effect, showing a higher rate of wound closure after 6 and 12 hours, even achieving 100% wound closure 24 hours earlier than the control group (Fig. 22B). Furthermore, the wound closure effect of the DHE formulation on hMSC showed a significantly higher wound healing rate at 48 hours compared to the control group;

however, complete wound closure was achieved at 72 hours in both treated and control conditions (Fig. 22C). These results indicate that DHE supplementation can significantly promote the wound healing rate, especially for hDFs.

### 2.3.4. Cellular bilaminar ACDHE hydrogel

Two types of cells were used for the biomanufacture of a bilayer hydrogel. On the one hand, hDFs, an essential component of the skin dermis, and on the other, hMSCs, which can provide growth factors, cytokines and chemokines that promote cell survival and regulate tissue regeneration. The ACDHE hydrogel formulation was prepared in a bilaminar manner with hDFs located in the top layer and hMSCs in the bottom layer. An hMSC-laden ACDHE layer was placed on the surface of a Petri dish and left to gel while the hDF-laden ACDHE solution was prepared. Then, the hDF-laden layer was added on the top of the hMSC-laden layer. Due to the viscosity of the ACDHE formulation, the two layers were not mixed during the process, but rather remained adhered during gelification. Once fully gelled, the samples were transferred to multiwell culture plates and immersed in cell culture medium. Fig. 23A shows the macroscopic appearance of the ACDHE bilayer hydrogel on days 0 and 21, showing that it could hold its shape over time.

To assess the distribution of cells within the hydrogels, hDFs and hMSCs were previously stained with Cell-TrackerTM Green CMFDA and CellTrackerTM Red CMTPX, respectively. Two well-differentiated layers could be observed with the two different cell types (Fig. 23B). The bottom view of the cross section made it possible to see the cells evenly distributed throughout the bilayer structure. Furthermore, this structure maintained its distribution, being able to observe both layers during the entire culture time. The cell viability images acquired on days 0, 7, 14, and 21 showed that most cells were viable, with few dead cells. The ACDHE bilayer hydrogel samples were shown to support cell viability for up to 21 days (Fig. 23C), keeping the clearly differentiated bilaminar distribution, with hDF showing a fibroblastic cell morphology (top) and hMSC showing a spherical shape (bottom). The proliferation rate of the bilaminar hydrogels on days 0, 1, 3, 5, 7, 10, 14, 18, and 21 showed an initial decrease in the proliferation rate (on days 1 to 5) and a subsequent significant increase (from day 10 to 21) (Fig. 23D).

Furthermore, the cell-laden ACDHE bilaminar hydrogels were also analysed using ESEM (Fig. 24). Encapsulated cells could be observed within the hydrogel matrix (Fig. 24A and B), and it even seemed that they could produce ECM-like structures (Fig. 24C and D). Figs. 24E and 24F show cells and deposited ECM.

### Claims

1. A monolaminar hydrogel comprising:

   - Type I collagen (Col I),
   - dermatan sulphate (DS),
   - hyaluronic acid (HA), and
   - agarose (Ag).

2. The monolaminar hydrogel according to claim 1, which further comprises elastin (EL).

3. A bilaminar hydrogel comprising two layers of the monolaminar hydrogel according to claim 2, wherein both layers comprise elastin.

4. The bilaminar hydrogel according to claim 3, comprising a first bottom layer comprising the monolaminar hydrogel according to claim 2, wherein said monolaminar hydrogel comprises mesenchymal stem cells (MSCs), and a second top layer, arranged on top of the first, comprising the monolaminar hydrogel according to claim 2, wherein said monolaminar hydrogel comprises dermal fibroblasts (DFs).

5. The bilaminar hydrogel according to claim 4, wherein the dermal fibroblasts and/or mesenchymal stem cells are human.

6. The bilaminar hydrogel according to any one of claims 3 to 5, wherein:

   - the top layer comprises Col I at a concentration from 1 to 3.5 mg/ml, DS at a concentration from 7 to 10 mg/ml, HA at a concentration from 0.5 to 1.5 mg/ml, Ag at a concentration from 0.010 to 0.030 mg/ml, and EL at a concentration from 0.5 to 1.5 mg/ml; and
   - the bottom layer comprises Col I at a concentration from 1 to 3.5 mg/ml, DS at a concentration from 7 to 10 mg/ml, HA at a concentration from 0.5 to 1.5 mg/ml, Ag at a concentration from 0.010 to 0.030 mg/ml, and EL at a concentration from 0.5 to 1.5 mg/ml.

7. The bilaminar hydrogel according to claim 6, wherein:

- the top layer comprises Col I at a concentration of 2.2 mg/ml, DS at a concentration of 8.4 mg/ml, HA at a concentration of 1 mg/ml, Ag at a concentration of 0.015 mg/ml, and EL at a concentration of 1 mg/ml; and
- the bottom layer comprises Col I at a concentration of 2.2 mg/ml, DS at a concentration of 8.4 mg/ml, HA at a concentration of 1 mg/ml, Ag at a concentration of 15 mg/ml, and EL at a concentration of 1 mg/ml.

8. A trilaminar hydrogel comprising:

- a bottom layer comprising the monolaminar hydrogel according to claim 1,
- a middle layer comprising the monolaminar hydrogel according to claim 1 or 2, and
- a top layer comprising a hydrogel comprising Col I, keratin (Kt), and sphingolipids (Sph).

9. The trilaminar hydrogel according to claim 8, which further comprises DFs, MSCs and/or epidermal keratinocytes (EKs).

10. The trilaminar hydrogel according to claim 9, wherein the top layer comprises EKs, the middle layer comprises DFs and the bottom layer comprises MSCs.

11. The trilaminar hydrogel according to claim 9 or 10, wherein the DFs, MSCs and/or EKs are human.

12. The trilaminar hydrogel according to any one of claims 8 to 11, wherein:

- the top layer comprises Col I at a concentration from 3.5 to 5.5 mg/ml, Kt at a concentration from 10 to 20 mg/ml and Sph at a concentration from 2.5 to 7.5 mg/ml;
- the middle layer comprises Col I at a concentration from 1 to 3.5 mg/ml, DS at a concentration from 7 to 10 mg/ml, HA at a concentration from 0.5 to 1.5 mg/ml, Ag at a concentration from 10 to 20 mg/ml, and EL at a concentration from 0.5 to 1.5 mg/ml; and
- the bottom layer comprises Col I at a concentration from 1 to 3.5 mg/ml, DS at a concentration from 7 to 10 mg/ml, HA at a concentration from 0.5 to 1.5 mg/ml, and Ag at a concentration from 10 to 20 mg/ml.

13. The trilaminar hydrogel according to claim 12, wherein:

- the top layer comprises Col I at a concentration of 4.4 mg/ml, Kt at a concentration of 15.2 mg/ml and Sph at a concentration of 5 mg/ml;
- the middle layer comprises Col I at a concentration of 2.2 mg/ml, DS at a concentration of 8.4 mg/ml, HA at a concentration of 1 mg/ml, Ag at a concentration of 15 mg/ml, and EL at a concentration of 1 mg/ml; and
- the bottom layer comprises Col I at a concentration of 2.2 mg/ml, DS at a concentration of 8.4 mg/ml, HA at a concentration of 1 mg/ml, and Ag at a concentration of 15 mg/ml.

14. The monolaminar hydrogel according to claim 1 or 2, the bilaminar hydrogel according to any one of claims 3 to 7, or the trilaminar hydrogel according to any one of claims 8 to 13, wherein the hydrogel is lyophilised.

15. A bio-ink comprising the hydrogel according to any one of claims 1 to 14.

16. Use of the hydrogel according to any one of claims 1 to 14 for the manufacture of a bio-ink.

17. Use of the hydrogel according to any one of claims 1 to 14, or of the bio-ink according to claim 15, for the manufacture of a dressing or implant, preferably cutaneous or subcutaneous.

18. A dressing or implant, preferably cutaneous or subcutaneous, comprising the hydrogel according to any one of claims 1 to 14, or the bio-ink according to claim 15.

19. A pharmaceutical composition comprising the hydrogel according to any one of claims 1 to 14, or the bio-ink according to claim 15.

**20.** The pharmaceutical composition, according to claim 19, which further comprises at least one active ingredient that favours the repair or regeneration of skin tissue.

**21.** The hydrogel according to any one of claims 1 to 14, the bio-ink according to claim 15, or the pharmaceutical composition according to claim 19 or 20, for use in medicine, preferably in regenerative medicine.

**22.** The hydrogel according to any one of claims 1 to 14, the bio-ink according to claim 15, or the pharmaceutical composition according to claim 19 or 20, for use in the regeneration, repair or replacement of skin tissue.

**23.** The hydrogel according to any one of claims 1 to 14, or the bio-ink according to claim 15, or the pharmaceutical composition according to claim 19 or 20, for use in the treatment of skin lesions or wounds.

**24.** Use of the hydrogel according to any one of claims 1 to 14, the bio-ink according to claim 15, the dressing according to claim 18, or the pharmaceutical composition according to claim 19 or 20, to promote *in vitro* wound healing.

**25.** Use of the hydrogel according to any one of claims 1 to 14, the bio-ink according to claim 15, the dressing according to claim 18, or the pharmaceutical composition according to claim 19 or 20, to promote *in vitro* melanin production in isolated skin cells.

**26.** *In vitro* use of the hydrogel according to any one of claims 1 to 14, the bio-ink according to claim 15, the dressing according to claim 18, to test drugs and/or toxic compounds for the skin.

**27.** A method for obtaining the hydrogel according to any one of claims 3 to 14, comprising the following steps:

a) mixing Col I, agarose, DS, HA and EL, obtaining a solution (i),
b) mixing Col I, agarose, DS, HA, obtaining a solution (ii), and
c) putting the solution (i) and the solution (ii) in contact, obtaining a bilaminar hydrogel.

**28.** The obtaining method according to claim 27, wherein step b) further comprises mixing EL with the rest of the components.

**29.** The obtaining method according to claim 27 or 28, wherein:

- the concentration of Col I is from 1 to 3.5 mg/ml, the concentration of DS is from 7 to 10 mg/ml, the concentration of HA is from 0.5 to 1.5 mg/ml, the concentration of Ag is from 10 to 30 mg/ml, and the concentration of EL is from 0.5 to 1.5 mg/ml in the solution (i); and
- the concentration of Col I is from 1 to 3.5 mg/ml, the concentration of DS is from 7 to 10 mg/ml, the concentration of HA is from 0.5 to 1.5 mg/ml, the concentration of Ag is from 10 to 30 mg/ml, and the concentration of EL is from 0.5 to 1.5 mg/ml in the solution (ii).

**30.** The obtaining method according to claim 29, wherein:

- the concentration of Col I is 2.2 mg/ml, the concentration of DS is 8.4 mg/ml, the concentration of HA is 1 mg/ml, the concentration of Ag is 15 mg/ml, and the concentration of EL is 1 mg/ml in the solution (i); and
- the concentration of Col I is 2.2 mg/ml, the concentration of DS is 8.4 mg/ml, the concentration of HA is 1 mg/ml and the concentration of Ag is 15 mg/ml in the solution (ii).

**31.** The obtaining method according to any one of claims 27 to 30, wherein Dermial ® is used as a source of DS and HA.

**32.** The obtaining method according to any one of claims 27 to 31, wherein step a) further comprises adding DFs, preferably human DFs (hDFs), and wherein step b) further comprises adding MSCs, preferably human MSCs (hMSCs).

**33.** The obtaining method according to any one of claims 27 to 32, which further comprises the following additional steps:

(d) mixing Col I with Kt and Sph, obtaining a solution (iii), and
(e) putting the solution (iii) in contact with the bilaminar hydrogel obtained in c), obtaining a trilaminar hydrogel.

34. The obtaining method according to claim 33, wherein the concentration in the solution (iii) of Col I is from 3.5 to 5.5 mg/ml, the concentration of Kt is from 10 to 20 mg/ml and the concentration of Sph is from 2.5 to 7.5 mg/ml.

35. The obtaining method according to claim 34, wherein the concentration in the solution (iii) of Col I is 4.4 mg/ml, the concentration of Kt is 15.2 mg/ml and the concentration of Sph is 5 mg/ml.

36. The obtaining method according to any one of claims 33 to 35, which further comprises adding EKs, preferably human EKs (hEKs).

37. The method for obtaining the hydrogel according to claim 36, wherein the EKs are added to the trilaminar hydrogel obtained in step (e).

38. The method for obtaining the hydrogel according to claim 36 or 37, wherein the added EKs are left to culture for 0.5 to 3 weeks, preferably 1 week.

39. The obtaining method according to any one of claims 27 to 38, which further comprises a step (f) of dehydrating the hydrogel.

40. The obtaining method according to any one of claims 27 to 39, which further comprises a final step of lyophilising the hydrogel.

41. The obtaining method according to any one of claims 27 to 40, wherein the agarose is previously heated to a temperature of 60 to 80°C, preferably 70°C, before mixing with the rest of the components in steps a) and b) of the method.

**Fig. 1**

Fig. 2

Fig. 3

Fig. 4

Fig. 5

Fig. 6

Fig. 7

Fig. 8

**Fig. 9**

Fig. 10

Fig. 11

Fig. 12

Fig. 13

Fig. 14

**Native Skin**

H&E  Masson's Trichrome

**Autograft**

H&E  Masson's Trichrome

**Control**

H&E  Masson's Trichrome

**Lyophilised**

H&E  Masson's Trichrome

Fig. 15

**Native Skin**

Fibronectin    Cytokeratin

**Control**

Fibronectin    Cytokeratin

**Autograft**

Fibronectin    Cytokeratin

**Lyophilised**

Fibronectin    Cytokeratin

Fig. 16

A    AC    AC$_{low}$

B

Fig. 17

Fig. 18

Fig. 19

Fig. 20

Fig. 21

Fig. 22

Fig. 23

Fig. 24

Europäisches
Patentamt

European
Patent Office

Office européen
des brevets

**EUROPEAN SEARCH REPORT**

Application Number

EP 23 38 2452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/112562 A1 (POSTECH RES & BUSINESS DEV FOUND [KR]) 10 June 2021 (2021-06-10) * paragraphs 11, 13; claims 1-9 * ----- | 1-41 | INV. A61L27/26 A61L27/38 A61L27/52 |
| X | NILLESEN S T M ET AL: "Design and in vivo evaluation of a molecularly defined acellular skin construct: Reduction of early contraction and increase in early blood vessel formation", ACTA BIOMATERIALIA, ELSEVIER, AMSTERDAM, NL, vol. 7, no. 3, 12 October 2010 (2010-10-12), pages 1063-1071, XP028131197, ISSN: 1742-7061, DOI: 10.1016/J.ACTBIO.2010.10.011 [retrieved on 2010-10-18] * section 2.2; caption of Figure 2 * ----- | 1-41 | A61L27/58 A61L27/60 B33Y80/00 |
| A | US 2011/150823 A1 (HUANG LYNN L H [TW]) 23 June 2011 (2011-06-23) * claims 1-5, 11; example 1 * ----- | 1-41 | |
| A | MASRI SYAFIRA ET AL: "Current Insight of Printability Quality Improvement Strategies in Natural-Based Bioinks for Skin Regeneration and Wound Healing", POLYMERS, vol. 13, no. 7, 25 March 2021 (2021-03-25), pages 1-22, XP093084688, DOI: 10.3390/polym13071011 * abstract * ----- -/-- | 1-41 | |

TECHNICAL FIELDS SEARCHED (IPC)

A61L
B33Y

The present search report has been drawn up for all claims

| Place of search | Date of completion of the search | Examiner |
|---|---|---|
| The Hague | 22 September 2023 | Cadamuro, Sergio |

EPO FORM 1503 03.82 (P04C01)

page 1 of 2

Europäisches
Patentamt
European
Patent Office
Office européen
des brevets

## EUROPEAN SEARCH REPORT

Application Number

EP 23 38 2452

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | P. SELCAN GUNGOR-OZKERIM ET AL: "Bioinks for 3D bioprinting: an overview", BIOMATERIALS SCIENCE, vol. 6, no. 5, 1 January 2018 (2018-01-01), pages 915-946, XP055708634, GB ISSN: 2047-4830, DOI: 10.1039/C7BM00765E * abstract * | 1-41 | |

-----

TECHNICAL FIELDS
SEARCHED (IPC)

The present search report has been drawn up for all claims

| Place of search | Date of completion of the search | Examiner |
|---|---|---|
| The Hague | 22 September 2023 | Cadamuro, Sergio |

EPO FORM 1503 03.82 (P04C01)

## ANNEX TO THE EUROPEAN SEARCH REPORT
## ON EUROPEAN PATENT APPLICATION NO.

EP 23 38 2452

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2023

| Patent document cited in search report | | Publication date | Patent family member(s) | | Publication date |
|---|---|---|---|---|---|
| WO 2021112562 | A1 | 10-06-2021 | KR 102263745 | B1 | 09-06-2021 |
| | | | WO 2021112562 | A1 | 10-06-2021 |
| US 2011150823 | A1 | 23-06-2011 | CN 102791254 | A | 21-11-2012 |
| | | | EP 2515867 | A2 | 31-10-2012 |
| | | | US 2011150823 | A1 | 23-06-2011 |
| | | | WO 2011087779 | A2 | 21-07-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106390205 A

- WO 2019040224 A1


**Non-patent literature cited in the description**

- **DAI C. et al.** Skin substitutes for acute and chronic wound healing: an updated review. *J Dermatologist Treat.,* 2020, vol. 31 (6), 639-648
- **LÓPEZ-RUIZ E et al.** *Eur Cells Mater.,* 2018, vol. 35, 209-24
- **GÁLVEZ-MARTÍN P et al.** *Eur J Pharm Biopharm.,* 2014, vol. 86 (3), 459-68

- **SIERRA-SÁNCHEZ Á et al.** *J Eur Acad Dermatology Venereol.,* 2020
- *Declaration of Helsinki of 1975,* 1983
- **CARRIEL V. et al.** *Cells Tissues Organs,* 2012, vol. 196196, 1-121
- **GÁLVEZ-MARTIN P et al.** *Eur J Pharm Biopharm.,* 2014, vol. 86 (3), 459-68